# EUROPEAN PATENT SPECIFICATION

(11) **EP 3 713 587 B1**
(45) Date of publication and mention of the grant of the patent: **27.03.2024**
(21) Application number: 18830137.8
(22) Date of filing: 26.11.2018
(51) Int. Cl.: A61K 35/745, A23L 33/135, A61P 1/00, A23L 33/00

(54) **A COMPOSITION COMPRISING A COHORT OF BACTERIA**
ZUSAMMENSETZUNG MIT EINER KOHORTE VON BAKTERIEN
COMPOSITION COMPRENANT UNE COHORTE DE BACTÉRIES

(30) Priority: 24.11.2017 EP 17203671; 29.11.2017 EP 17204531
(43) Date of publication of application: 30.09.2020
(73) Proprietor: University College Cork-National University of Ireland, Cork, Cork (IE); Agriculture and Food Development Authority (TEAGASC), Co. Carlow (IE)
(72) Inventor: STANTON, Catherine, Cork Kilworth (IE); ROSS, Paul, Cork Kilworth (IE); FITZGERALD, Ger, Cork (IE); RYAN, Tony, Cork (IE)
(74) Representative: Purdylucey Intellectual Property
(86) International application number: PCT/EP2018/082615
(87) International publication number: WO 2019/102018

(56) References cited:
- WO-A1-2013/144701
- US-A- 5 711 977
- A. SKÓRKA ET AL: "To add or not to add probiotics to infant formulae? An updated systematic review", BENEFICIAL MICROBES, vol. 8, no. 5, 13 October 2017 (2017-10-13), pages 717-725, XP055471204, NL ISSN: 1876-2883, DOI: 10.3920/BM2016.0233
- S. DELGADO ET AL: "In vitro evaluation of the probiotic properties of human intestinal Bifidobacterium species and selection of new probiotic candidates", JOURNAL OF APPLIED MICROBIOLOGY., vol. 104, no. 4, 1 April 2008 (2008-04-01) , pages 1119-1127, XP055471189, GB ISSN: 1364-5072, DOI: 10.1111/j.1365-2672.2007.03642.x
- BARRETT EOIN ET AL: "The neonatal gut harbours distinct bifidobacterial strains.", ARCHIVES OF DISEASE IN CHILDHOOD. FETAL AND NEONATAL EDITION SEP 2015, vol. 100, no. 5, September 2015 (2015-09), pages F405-F410, XP002780625, ISSN: 1468-2052
- SILVIA ARBOLEYA ET AL: "Gene-trait matching across the pan-genome reveals considerable diversity in carbohydrate catabolism among human infant strains", BMC GENOMICS, BIOMED CENTRAL LTD, LONDON, UK, vol. 19, no. 1, 8 January 2018 (2018-01-08), pages 1-16, XP021252288, DOI: 10.1186/S12864-017-4388-9
- BARRETT EOIN ET AL: "The neonatal gut harbours distinct bifidobacterial strains.", ARCHIVES OF DISEASE IN CHILDHOOD. FETAL AND NEONATAL EDITION SEP 2015, vol. 100, no. 5, September 2015 (2015-09), pages F405-F410, ISSN: 1468-2052

## Description

### Field of the Invention

The present invention relates to compositions comprising a cohort of bacteria. The invention also relates to strains of *Bifidobacterium longum* which have utility in improving or maintaining gut health.

### Background to the Invention

Currently, the vast majority of probiotic products on the market comprise of single strains or a small number of strains belonging to distinct species. These products are formulated based on probiotic effects of the strains in the formulation, for example promoting a healthy digestive tract, a health immune system, or more specific indications such as preventing weight gain or modulating blood glucose levels. These products work on the basis of a supplementation strategy, where the microbiome is supplemented with a probiotic bacterium that is known to elicit a specific beneficial effect. Some patient groups exhibit a dysregulated microbiome in which dominant species of bacteria in the microbiome are absent or present in reduced numbers, leading to gut-related health issues. An example is the microbiome of infants that are premature or have been treated with antibiotics. C-section delivery of infants has recently been associated with decreased colonization rates of Bifidobacterium, Bacteroides and Lactobacillus, with a decrease in diversity and richness of the microbiota. It has also been associated with an increased risk of developing obesity, type 1 diabetes, as well as immune disorders such as asthma or allergies. In these patients, treatment with specific probiotic bacteria, or cohorts of probiotic bacteria from distinct species, is unlikely to confer a beneficial effect due to the complexity and diversity of the microbiome. Moreover, most probiotic strains that are administered orally do not persist in the gut and are excreted.

WO2013/144701 describes compositions comprising strains of *Bifidobacterium longum.*

It is an object of the invention to overcome at least one of these problems.

### Summary of the Invention

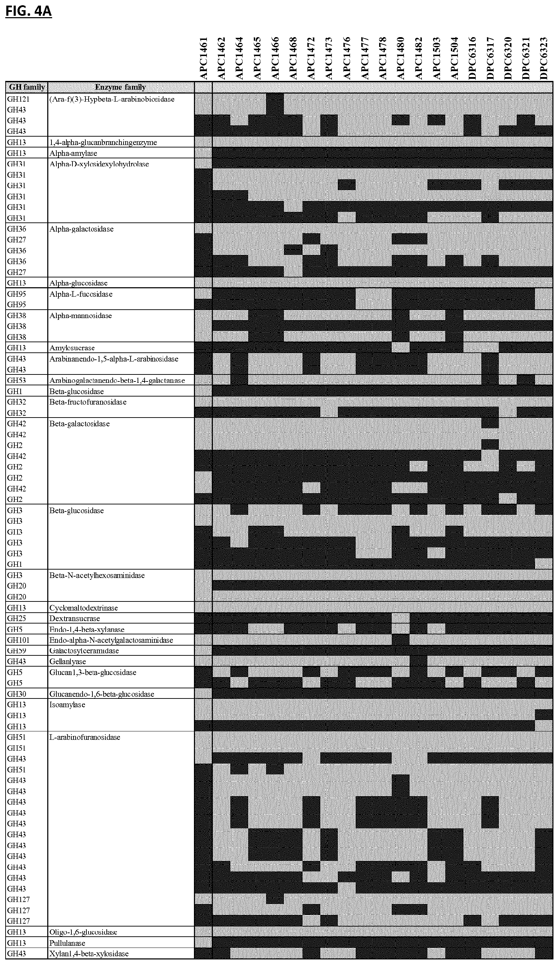

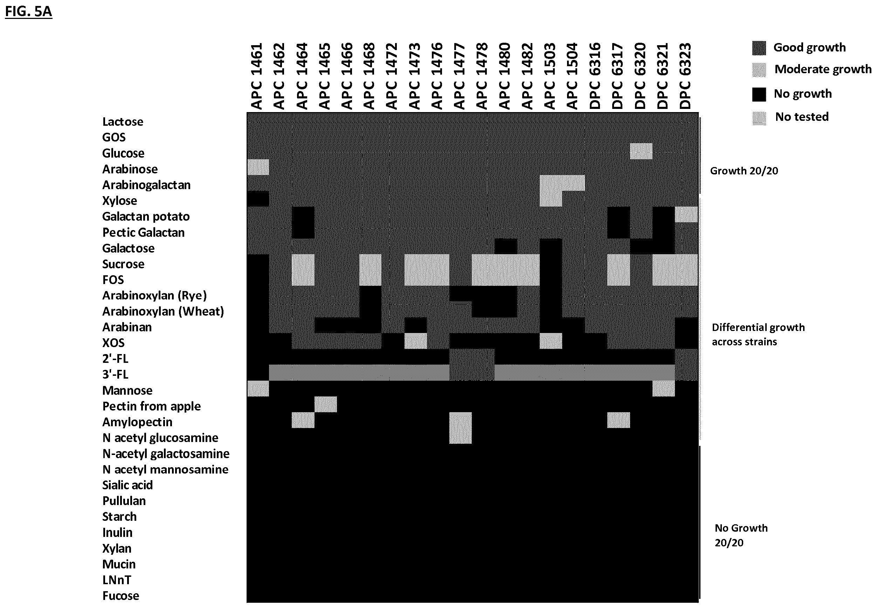

In one aspect, the invention provides a composition as outlined in the appended claims. The plurality of isolated strains of *Bifidobacterium longum* bacteria (Table 1) that display a broad range of complex carbohydrate utilisation (Fig. 5A) and a broad range of glycosyl hydrolase expression (Fig. 4A). Each of the strains of Table 1 also comprises 30% of the *Bifidobacterium longum* pangenome. Compositions comprising a plurality of these strains - for example at least 5 isolated strains - will exhibit different and diverse catabolic capability thereby increasing the chances that one or more of the strains in the composition will find a nutritional niche in the infant GI tract and so would persist and help restore the composition of the microbiota to normal. As an example, and referring to Fig. 5A, the following cohorts of strains of Table 1 are capable of utilising lactose, GOS, glucose, arabinose, arabinogalactan, xylose, galactan potato, pectic galactan, galactose, sucrose, FOS, arabinoxylan (rye and wheat), arabinan, XOS, and at least one of the human milk oligosaccharides 2'-fucosyllactose (2'-FL) and 3'-fucosyllactose (3'-FL):
Cohort 1 - APC 1472, 1473, 1476, 1478 and 1480;
Cohort 2 -APC 1465, 1466, 1468, 1472 and 295 (DPC6323); and
Cohort 3 -APC 1461, 1462, 1464, 1465 and 1466.

Two of the strains of Table 1 exhibit good growth in the human milk oligosaccharides 2'-fucosyllactose and 3'-fucosyllactose (APC 1478 and APC 296 (DPC6326)) - Figs 4A and 5A, which indicates that these strains would find a nutritional niche in an infant GI tract as the infant would be ingesting the oligosaccharises through mothers milk or infant formula. The invention therefore also relates to compositions comprising a cohort of isolated B. *longum* strains of Table 1 that include at least one or both of strains APC 1478 and APC 295 (DPC6323). The invention therefore also relates to compositions comprising an isolated *B. longum* strain selected from strains APC 1477, APC 1478 and APC 295 (DPC6323).

The compositions of *B. longum* strains of the invention may therefore be administered to subjects that exhibit a dysregulated microbiome, especially subjects the exhibit a *B. longum* deficit such as infants in the first six months who are pre-mature, born by caesarean section, or have undergone or are undergoing antibiotic therapy, to increase the diversity and richness of the microbiota and avoid adverse health effects associated with dysregulated microbiome.

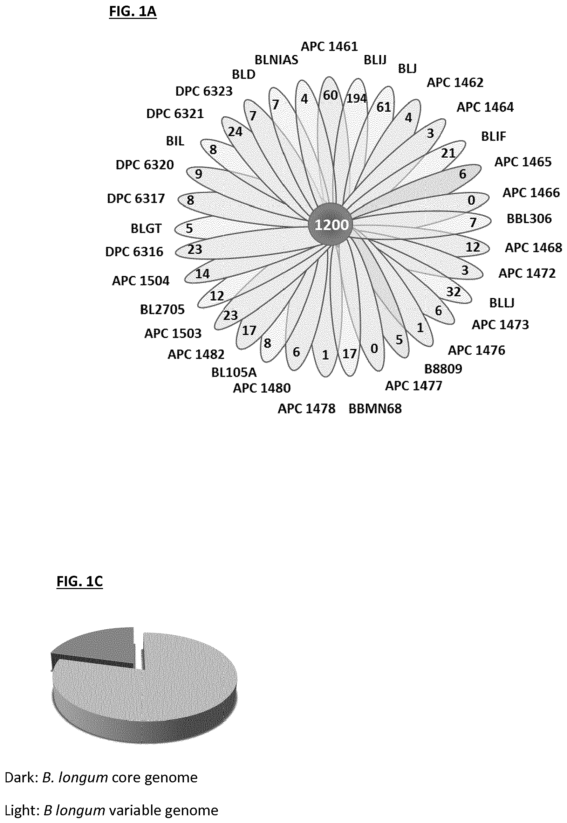

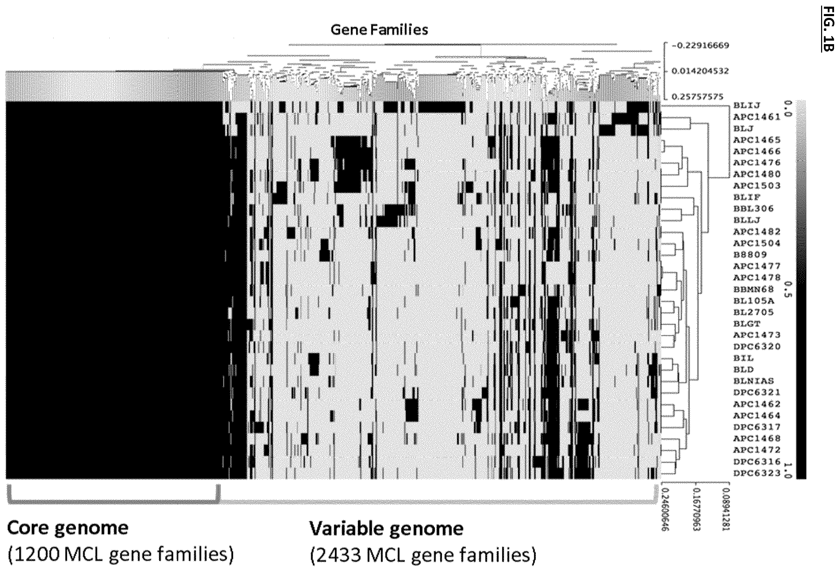

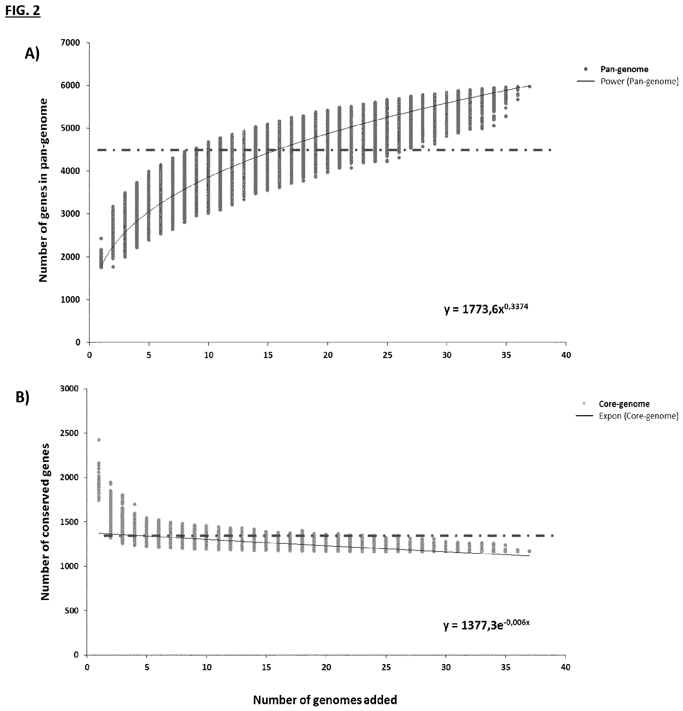

In another aspect, the present invention provides a composition as outlined in the appended claims comprising a cohort of unique strains all of which belong to the same species, in which the composite genetic repertoire of the strains in the cohort (i.e. the number of ORF's in the cohort of strains) is representative of the pangenome of the species, for example at least 60%, 70% or 80% of the pangenome. Compared with single strains, or cohorts of strains of distinct species, the composition of the invention confers improved health effects due to the diversity and complexity of the microbial population in the human gut, especially in patients having a dysregulated microbiome, and is based on a *species-replacement* strategy as opposed to a *strain-supplementation* strategy. In one embodiment, the invention is directed to a composition comprising a cohort of unique *Bifidobacterium longum* strains having a composite genetic repertoire that is representative of the *Bifidobacterium longum* pangenome (i.e. represents at least 60% of the pangenome) , and the use of this composition in subjects where *Bifidobacterium longum* is known to be a dominant member of the microbiome, for example infants, and especially infants that have a dysregulated or immature microbiome such as premature infants, infants born by C-section or infants that have been treated with antibiotics. Each of the strains of Table 1 comprises 30% of the *Bifidobacterium longum* pangenome, and it is a routine task for a person skilled in the art to pick a cohort of isolated strains from Table 1 that together represent at least 60% of the *Bifidobacterium longum* pangenome using the information provided herein, in particular Figs 1 and 2. All of the strains of Table 1 represent about 80% of the *Bifidobacterium longum* pangenome.

The polybiotic composition of the invention is also applicable in individuals that have a dysregulated microbiome due to depletion of other bacterial species, and may be employed to supplement or replenish the microbiome in the affected individual to overcome the microbiome deficit, and re-store the microbiome to a healthy and fully functional state. In many cases, the microbiome dysregulation involves severe depletion of a species of bacteria that is a dominant member of the gut microbiome (for example, *B. longum* in infant humans). Examples of individuals with a dysregulated microbiome include sub-groups of the population, such as elderly individuals, individuals undergoing antibiotic and chemo therapy, individuals that have suffered trauma to the gut (for example due to gut surgery or resection, or gut injury), or individuals that have disease that causes a dysregulation to the gut microbiome. Other sub-groups include pre-term infants and autism patients (microbiome depleted in *Bifidobacteria*)*.*

According to an aspect of the present invention, there is provided a composition of Claim 1. The composition comprises a cohort of at least five isolated unique strains of a single species of bacteria, wherein the species of bacteria is represented in the mammalian microbiome, and in which the cohort of isolated strains typically comprises a composite genetic profile that is representative of a pangenome of the species.

In one embodiment, the species of bacteria is represented in the human microbiome.

In one embodiment, the species of bacteria is a dominant member of the mammalian or human microbiome. In one embodiment, all or substantially all of the strains are represented in the mammalian gut. In one embodiment, all or substantially all of the strains are represented in the human gut. In one embodiment, all or substantially all of the strains are represented in the infant human gut. In one embodiment, all or substantially all of the strains are represented in the adult human gut. Typically all of the bacteria in the cohort are probiotic bacteria.

In one embodiment, the cohort of isolated strains comprises at least 6 or 8 isolated strains of the single species of bacteria.

In one embodiment, the cohort of isolated strains comprises at least 10, 12, 14, 16, 18 or 20 isolated strains of the single species of bacteria.

In one embodiment, each strain is capable of survival in a simulated intestinal environment.

In one embodiment, the species of bacterium is dominant in the gut of a sub-set of mammals characterised by common phenotype. In one embodiment, the common phenotype is selected from one of age, sex and ethnicity.

In one embodiment, the cohort of strains of bacteria exhibit a variation in sugar utilisation profiles.

In one embodiment, the species of bacterium is present in the gut of a healthy mammal and absent or in lesser amounts in the gut of a mammal having an abnormal pathology.

In one embodiment, the species of bacteria is a *Bifidobacterium,* for example *Bifidobacterium longum, Bifidobacterium breve, and Bifidobacterium bifidum.*

In one embodiment, the species of bacteria is *Bifidobacterium longum.*

In one embodiment, the strains of *Bifidobacterium longum* are all of human infant intestinal origin.

In one embodiment, the cohort of strains of bacteria predominantly comprises *Bifidobacterium longum ssp. Longum* strains.

In one embodiment, the cohort of strains of bacteria comprises at least five strains selected from Table 1 below:

**TABLE 1**

| | |
|---|---|
| *Bifidobacterium longum* ssp. *suis* APC | 1461 ((NCIMB 42832) |
| *Bifidobacterium longum* ssp. *longum* APC | 1462 (NCIMB 42833) |
| *Bifidobacterium longum* ssp. *longum* APC | 1464 (NCIMB 42839) |
| *Bifidobacterium longum* ssp. *longum* APC | 1465 (NCIMB 42834) |
| *Bifidobacterium longum* ssp. *longum* APC | 293** (NCIMB 42830) |
| *Bifidobacterium longum* ssp. *longum* APC | 295* (NCIMB 42831) |
| *Bifidobacterium longum* ssp. *longum* APC | 1468 (NCIMB 42840) |
| *Bifidobacterium longum* ssp. *longum* APC | 1473 (NCIMB 42824) |
| *Bifidobacterium longum* ssp. *longum* APC | 1476 (NCIMB 42825) |
| *Bifidobacterium longum* ssp. *longum* APC | 1478 (NCIMB 42826) |
| *Bifidobacterium longum* ssp. *longum* APC | 1504 (NCIMB 42841) |
| *Bifidobacterium longum* ssp. *longum* APC | 1480 (NCIMB 42827) |
| *Bifidobacterium longum* ssp. *longum* APC | 1503 (NCIMB 42828) |

| | |
|---|---|
| * APC 295 referenced in the Figures as DPC 6323 ** APC 293 referenced in the Figures as DPC 6321 | |

All of the above strains are characterised to species level by 16s rRNA-internally transcribed spacer (ITS) gene sequence analysis and to strain level by pulsed field gel electrophoresis (PFGE). The strains were all isolated from faecal samples from healthy, human infants. All strains were culturable. The strains were confirmed as unique due to distinct PFGE profiles and varying phenotypic traits.

In one embodiment, the cohort of strains comprises a selection of strains from Table 1 that together represent at least 65%, 70%, 75%, 80%, 85% or 90% of the *Bifidobacterium longum* pangenome. In one embodiment, the cohort of isolated strains includes at least one of APC 1478 and APC 295. In one embodiment, the cohort of isolated strains includes APC 1478 and APC 295. In one embodiment, the cohort of isolated strains includes at least one of APC 1478. APC 1477 and APC 295. In one embodiment, the cohort of isolated strains includes at least two of APC 1478. APC 1477 and APC 295. In one embodiment, the cohort of isolated strains includes all of APC 1478. APC 1477 and APC 295.

In one embodiment, the cohort of isolated strains includes at least one of APC 1478 and APC 295, and four additional isolated strains from Table 1, and in which the cohort of isolated strains comprises a composite genetic profile that corresponds with at least 60% of the *Bifidobacterium longum* pangenome. In one embodiment, the cohort of isolated strains includes APC 1478 and APC 295, and four additional isolated strains from Table 1, and in which the cohort of isolated strains comprises a composite genetic profile that corresponds with at least 60% of the *Bifidobacterium longum* pangenome. In one embodiment, the cohort of isolated strains includes APC 1478, APC 1477 and APC 295, and three additional isolated strains from Table 1, and in which the cohort of isolated strains comprises a composite genetic profile that corresponds with at least 60% of the *Bifidobacterium longum* pangenome.

In one embodiment, the cohort of strains is selected from:
Cohort 4: APC 1478, 295, 1462, 1465, 1466, 1472
Cohort 5: APC 1478, 295, 1462, 1465, 1466, 1476
Cohort 6 APC 1465, 1466, 1478, 1480, 1503, 1462
Cohort 7: APC 1465, 1466, 1478, 1480, 1503, 1464
Cohort 8: APC 1465, 1466, 1476, 1480, 1503, 1477

In one embodiment, the cohort of strains of bacteria comprises all or substantially all of the strains of Table 1.

In one embodiment, the cohort of strains of bacteria consists essentially of *the strains of Table 1.*

In one embodiment, the cohort of isolated strains comprises at least 5 or 6 strains selected from the strains of Table 1 and Table 2:

**TABLE 2**

| | |
|---|---|
| *Bifidobacterium longum* APC | 1466 |
| *Bifidobacterium longum* APC | 1472 (NCIMB 42795) |
| *Bifidobacterium longum* DPC | 6316 |
| *Bifidobacterium longum* DPC | 6320 |
| *Bifidobacterium longum* APC | 1477 |

In one embodiment, the cohort of strains comprises at least 10, 11, 12, 13, 14, 15, 16, 17, 18, 19 or 20 strains of Table 1 and 2.

The composition may be provided in a unit dose form suitable for oral administration, i.e. a tablet or capsule. The composition may be a food or beverage product, or a nutritional supplement. The composition may comprise a probiotic material. The composition may comprise a prebiotic material. The composition may comprise an additional probiotic bacterium. The strains in the composition may be viable or non-viable. The composition may comprise at least 10⁶ cfu per gram of composition.

The composition may be solid or liquid. The composition may comprise a carrier for oral delivery. The carrier may be in the form of tablet, capsule, powder, granules, microparticles or nanoparticles. The carrier may be configured for targeted release in the intestine (i.e. configured for gastric transit and ileal release by for example microencapsulation). The carrier may be configured for controlled release in the intestine (i.e. configured for gastric transit and ileal release).

The composition may be dried or lyophilised.

In a further aspect, the invention provides a composition comprising a cohort of at least five isolated strains of a single species of bacteria, wherein the species of bacteria is represented in the mammalian microbiome, and in which the cohort of isolated strains exhibits a variation in sugar utilisation profile. In one embodiment, the species is a probiotic species, typically a human probiotic species. In one embodiment, the cohort of isolated strains is capable of utilising a number of different sugars including human milk oligosaccharides and preferably plant derived carbohydrates. In one embodiment, the cohort of strains is together capable of utilising 3, 4 or all of xylo-oligosaccharides, arabinan, arabinoxylan, galactan, and fucosyllactose. In one embodiment, the cohort of strains is together capable of utilising substantially all of lactose, GOS, glucose, arabinose, arabinogalactan, xylose, galactan potato, pectic galactan, galactose, sucrose, FOS, arabinoxylan (rye and wheat), arabinan, XOS, and at least one or both of the human milk oligosaccharides 2'-fucosyllactose (2'-FL) and 3'-fucosyllactose (3'-FL). In one embodiment, the cohort of strains is capable of utilising the human milk oligosaccharides 2'-fucosyllactose (2'-FL) and 3'-fucosyllactose (3'-FL). In one embodiment, at least 2 or 3 of the cohort of strains is capable of utilising the human milk oligosaccharides 2'-fucosyllactose (2'-FL) and 3'-fucosyllactose (3'-FL).

In a further aspect, there is provided an infant formula product for human infants comprising a composition of the invention. Typically, the species of bacteria is *Bifidobacterium,* preferably *Bifidobacterium longum.*

In a further aspect, the invention provides a pharmaceutical or probiotic composition comprising a composition of the invention and a suitable pharmaceutical excipient.

The composition may be provided in a unit dose form suitable for oral administration, i.e. a tablet or capsule.

In a further aspect, the invention provides a composition of the invention, for use as a medicament.

In a further aspect, the invention provides a composition of the invention, for use in a method of treating a subject characterised by a having a gut microbiome that is deficient in a species of gut bacteria that is normally present in the gut of the healthy subject, wherein the composition comprises a cohort of strains of the species of bacteria that is deficient, especially *Bifidobacterium longum.* The composition may be used to treat or prevent elderly patients, pre-term infants, infants born by Caesarean section, infants undergoing or having undergone antibiotic therapy or another therapeutic regime that dysregulated the mirobiome in a subject, autism, metabolic disease, patients undergoing antibiotic or chemo-therapy. The method may be employed to help re-populate the subjects microbiome with the deficient specifies of bacteria, and help restore a normal microbiota in the subject. Restoration of a normal microbiota in an infant helps with normal development of the infant, and helps avoid conditions associated with dysregulated microbiota such as obesity, type 1 diabetes, as well as immune disorders such as asthma or allergies.

In one embodiment, the mammal is a human infant, or a pre-term infant, or an infant born by Caesarean section, or an autism patient, and the composition comprises a cohort of strains of *Bifidobacterium longum.*

Although not part of the current claims, a method of formulating a composition according to the invention is disclosed, the method comprising the steps of:
identifying a cohort of strains of a species of bacteria that comprises a composite genetic profile that is representative of a pangenome of the species; and
formulating a composition comprising the cohort of strains suitable for oral administration.

In one embodiment, the step of identifying the cohort of strains involves a step of identifying strains capable of survival in a simulated intestinal environment.

In one embodiment, the step of identifying the cohort of strains involves a step of determining the sugar utilisation profile of the strains.

In one embodiment, the composition is formulated for gastric transit and ileal release.

Also disclosed is an isolated bacterium selected from Table 1 or 2.

In one embodiment, the isolated bacterium is culturable. In one embodiment, the bacterium is derived from a human intestine, especially a human infant intestine. In one embodiment, the bacterium is capable of survival in a simulated intestinal environment. In one embodiment, the strain of the invention is a wild-type strain. In one embodiment of the invention, the strain is genetically modified.

In a further aspect, the invention provides a composition comprising five or more of the strains of bacteria of the invention, for example 5, 6, 7, 8, 9, 10, 11, 12, 13 or 14 In a further aspect, there is provided an infant formula product for human infants comprising an isolated bacterium of the invention.

In a further aspect, the invention provides a pharmaceutical or probiotic composition comprising an isolated bacterium of the invention, and a suitable pharmaceutical excipient. In a further aspect, the invention provides an isolated bacterium of the invention, for use as a medicament.

Table 1 and 2 discloses isolated bacterium. The composition of the invention may be an infant formula. In a further aspect, the invention provides the composition of the invention for use in a method of treating a subject characterised by a having a gut microbiome that is deficient in a species of gut bacteria that is normally present in the gut of the healthy subject. The composition may be used to treat or prevent elderly patients, pre-term infants, infants born by Caesarean section, infants undergoing or having undergone antibiotic therapy or another therapeutic regime that dysregulated the mirobiome in a subject, autism, metabolic disease, patients undergoing antibiotic or chemo-therapy. The method may be employed to help re-populate the subjects microbiome with the deficient specifies of bacteria, and help restore a normal microbiota in the subject. Restoration of a normal microbiota in an infant helps with normal development

Other aspects and preferred embodiments of the invention are defined and described in the other claims set out below.

### Brief Description of the Figures

**Figure 1****:** A) Venn diagram displaying core gene families obtained by MCL clustering, and unique genes of B. longum APC/DPC strains and B. longum complete genomes. B) Hierarchical clustering heatmap representing the variability of B. longum in terms of presence/absence of gene families. C) Pie chart indicating the percentage of variable and core with respect to the total of gene families, resulting from the MCL clustering algorithm. LEGEND: APC 1461: B. longum APC 1461; APC 1462: B. longum APC 1462; APC 1464: B. longum APC 1464; APC 1465: B. longum APC 1465; APC 1466: B. longum APC 1466; APC 1468: B. longum APC 1468; APC 1472: B. longum APC 1472; APC 1473: B. longum APC 1473; APC 1476: B. longum APC 1476; APC 1477: B. longum APC 1477; APC 1478: B. longum APC 1478; APC 1480: B. longum APC 1480; APC 1482: B. longum APC 1482; APC 1503: B. longum APC 1503; APC 1504: B. longum APC 1504; BLIJ: B. longum ssp. infantis ATCC15697; BLJ: B. longum ssp. longum JDM301; BLIF: B. longum ssp. longum 157F; BBL306: B. longum ssp. longum CCUG30698; BLLJ: B. longum ssp. longum JCM1217; B8809: B. longum ssp. longum NCIMB8809; BBMN68: B. longum ssp. longum BBMN68; BL105A: B. longum ssp. longum 105A; BL2705: B. longum ssp. longum NCC2705; BLGT: B. longum ssp. longum GT15; BIL: B. longum ssp. longum F8; BLD: B. longum ssp. longum DJO10A; BLNIAS: B. longum ssp. longum KACC9156; DPC 6316: B. longum DPC 6316; DPC 6317: B. longum DPC 6317; DPC 6320: B. longum DPC 6320; DPC 6321: B. longum DPC 6321; DPC 6323: B. longum DPC 6323.
**Figure 2****:** Pan-genome and core-genome of B. longum. A) The pan-genome plot is represented by the accumulated number of new genes against the number of genomes added. B) The core-genome plot is represented by the accumulated number of genes attributed to the core-genomes against the number of added genomes. The deduced mathematical function is reported. The red line represents the pan-genome and core-genome attribute to the twenty B. longum APC/DPC strains.

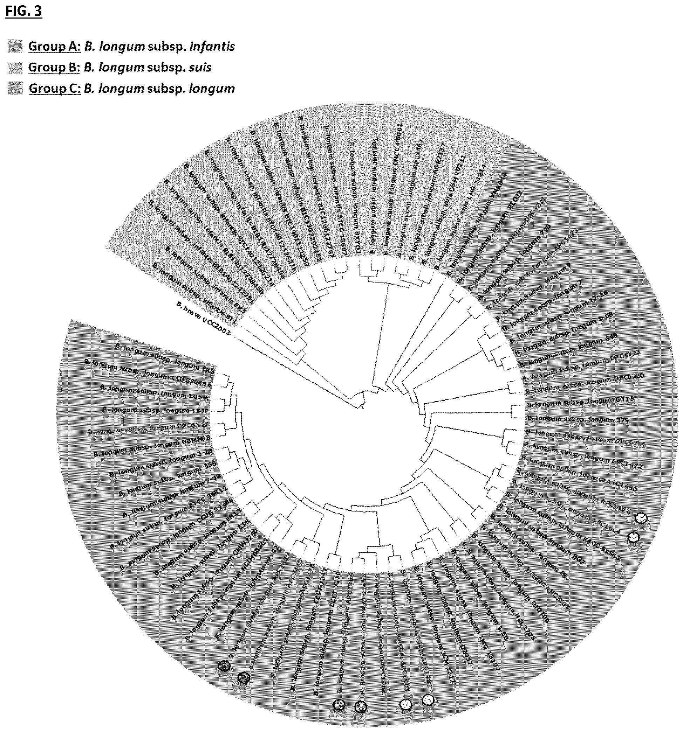

**Figure 3****:** Phylogenetic analysis of B. longum. Phylogenetic supertree showing the relationship between seventy-three complete and incomplete B. longum strains and B. breve UCC 2003 as an outlier. B. longum APC/DPC strains are coloured in red. Grey circles with different fillings represent isolated from the same infant.

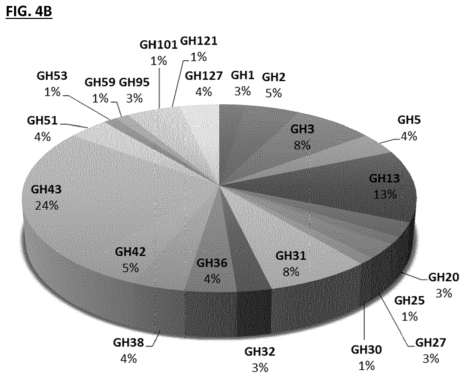

**Figure 4****:** Glycobiome. A) Heatmap displaying the in-silico prediction of the GH family members identified in the B. longum genomes (Table 6). B) Pie chart indicating the percentage of each GH families identified only in B. longum APC/DPC genomes.

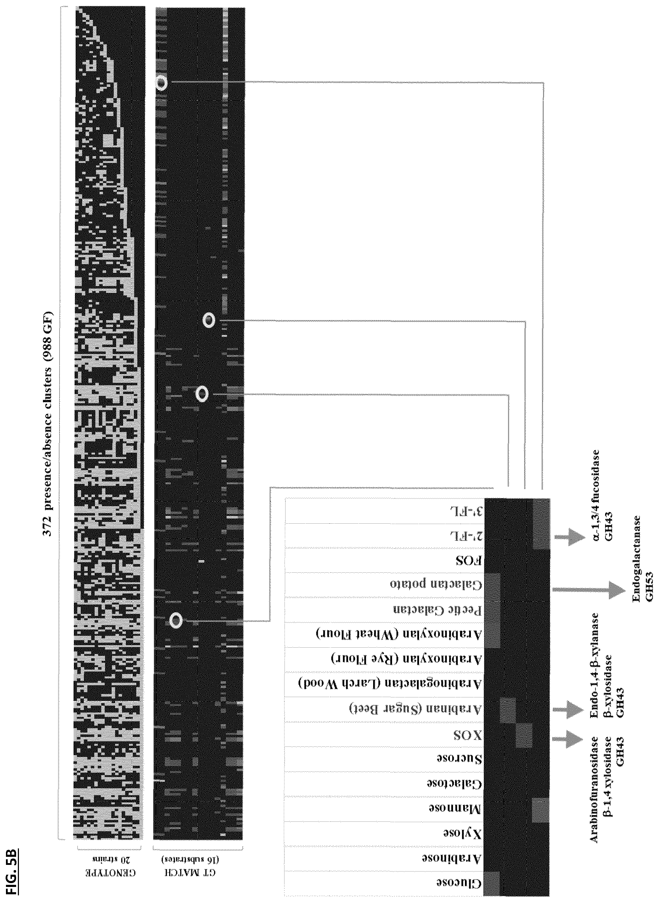

**Figure 5****:** Evaluation of carbohydrate utilization by B. longum strains. A) Heatmap showing the growth performance of B. longum APC/DPC strains on different carbon sources at 12 hours. B) Heatmap displaying the in-silico gene-trait matching exercise performed based on the association between the presence/absence of GH families predicted and the growth/no growth phenotype of the B. longum APC/DPC strains.

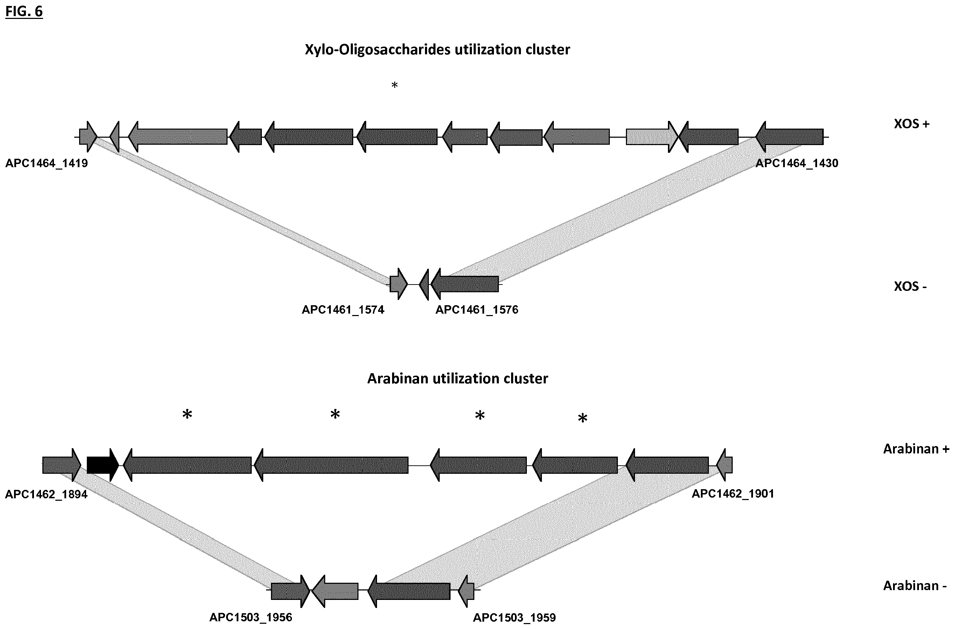

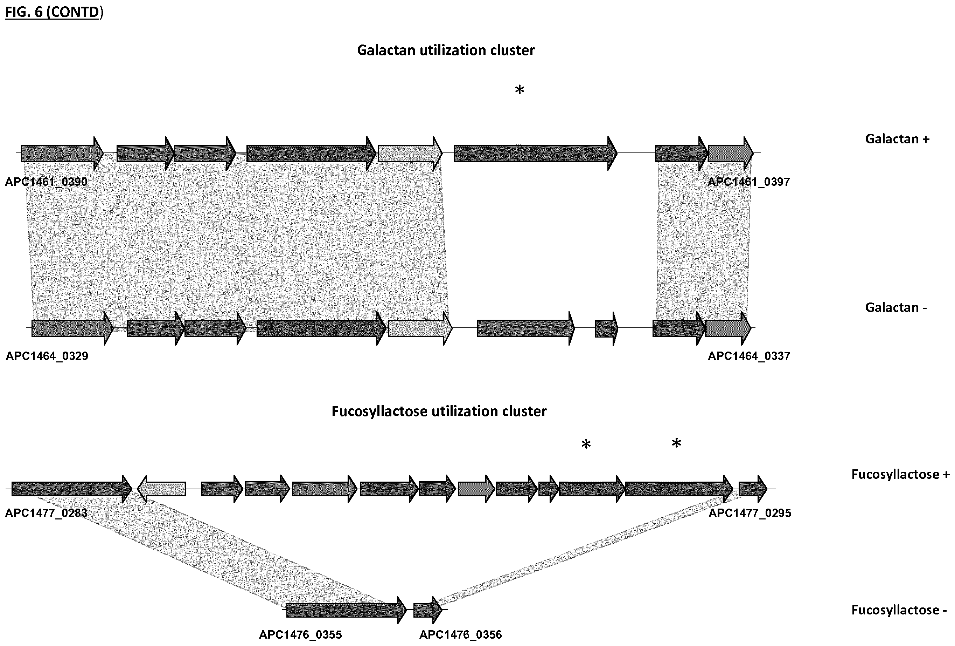

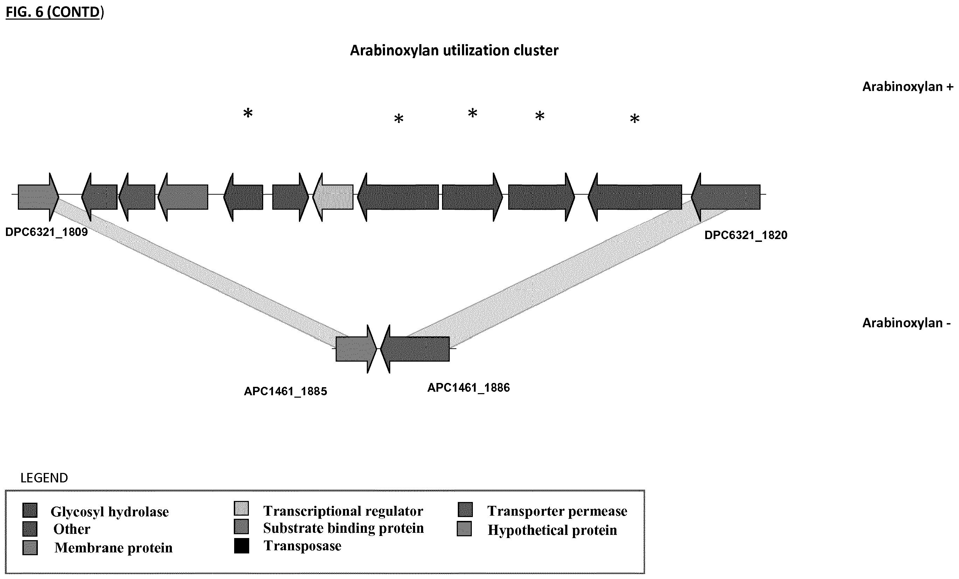

**Figure 6****:** Carbohydrates clusters predicted by GTM. Locus map showing the gene cluster putatively involved in the utilization of the different sugars in certain B. longum strains positive and negative for these carbohydrates.

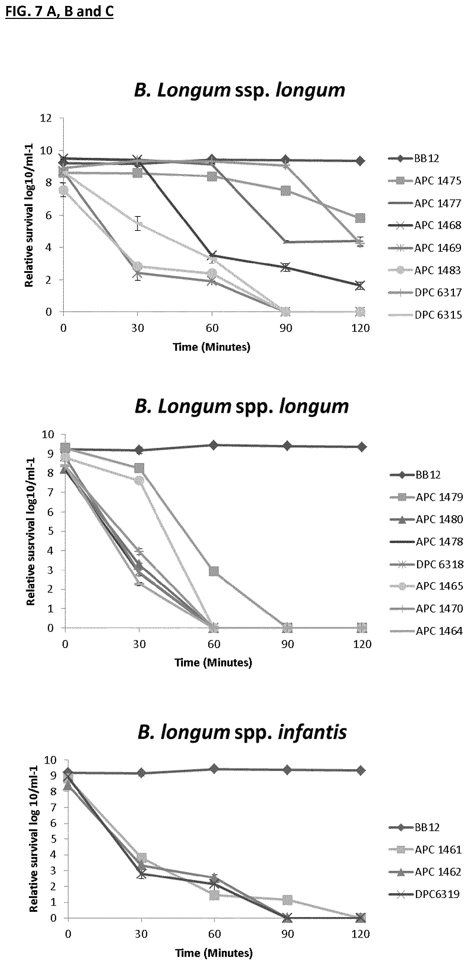

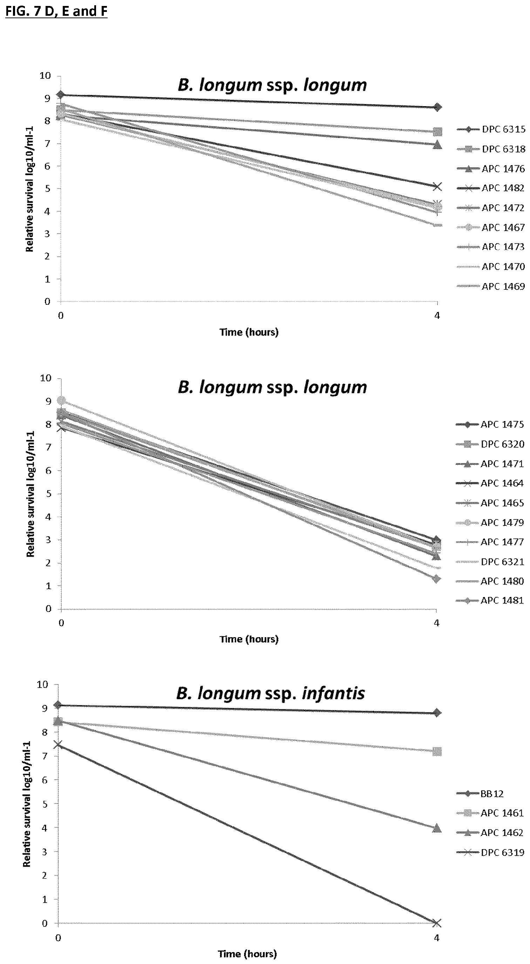

**Figure 7****:** Viability of B. longum strains in pH 2.5 and comparison with reference strain B. animalis subsp. lactis (BB-12). The data are means of duplicate experiments where the error bars indicate SEM (d-f) Viability of B. longum strains and comparison with reference strain B. animalis subsp. lactis (BB-12) in the presence of 0.3 % (w/v) bovine bile.

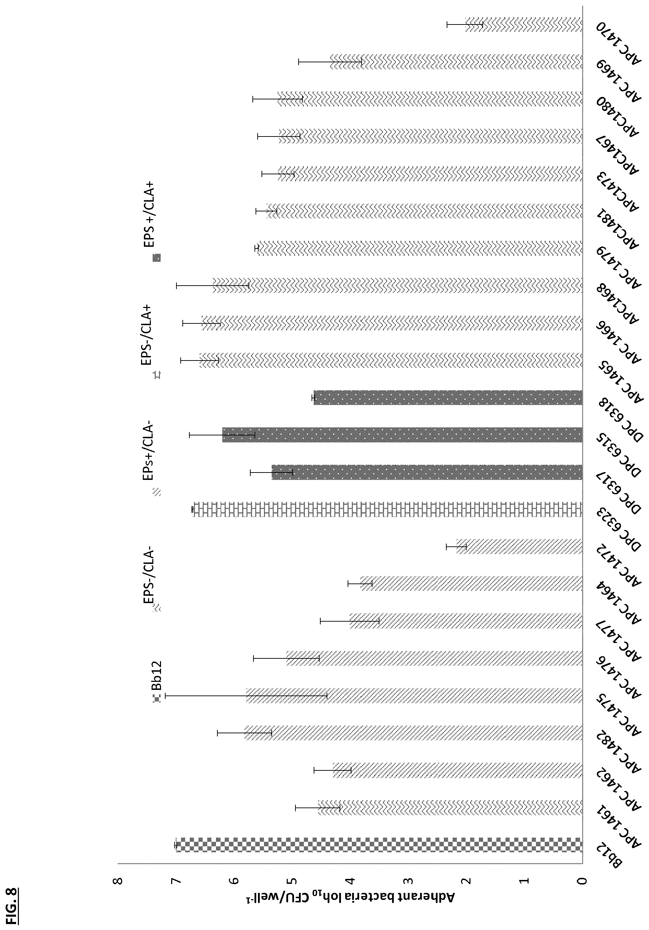

**Figure 8**:_Adhesion of B. longum strains and comparison with reference strain B. animalis subsp. lactis (BB-12). Adhesion is expressed as log CFU bacterial cells that bound to HT-29 cells within each well (2×105). Error bars represent SEM.

### Detailed Description of the Invention

### Definitions and general preferences

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and *vice versa.* The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g. a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or open-ended and does not exclude additional, unrecited integers or method/process steps.

### Treatment

As used herein, the term "disease" is used to define any abnormal condition that impairs physiological function and is associated with specific symptoms. The term is used broadly to encompass any disorder, illness, abnormality, pathology, sickness, condition or syndrome in which physiological function is impaired irrespective of the nature of the aetiology (or indeed whether the aetiological basis for the disease is established). It therefore encompasses conditions arising from infection, trauma, injury, surgery, radiological ablation, poisoning or nutritional deficiencies. The strains of the invention may be employed to treat or prevent disease or conditions, for example diseases or conditions characterised by a dysregulated microbiome, and other diseases or conditions including metabolic disease (for example Type I or Type II diabetes), inflammatory disease, cardiovascular disease, proliferative diseases, autoimmune disease, or degenerative conditions.

As used herein, the term "dysregulated microbiome" as applied to a mammal, for example a human, for example an infant human, should be understood to mean a microbiome that is depleted in one or more species of bacteria normally present in the microbiome. An example is the microbiome of an infant (i.e. up to six months old) that was born prematurely, born by Caesarian section, or has been treated with antibiotics, and which has a microbiome which is depleted in *Bifidobacterium longum* strains of bacteria. Other examples include elderly patients, patients undergoing antibiotic or chemo-therapy, autism patients, all of whom are recognised as having a microbiome depleted in *Bifidobacteria* and would be suitable for treatment with a composition of the invention.

As used herein, the term "treatment" or "treating" refers to an intervention (e.g. the administration of an agent to a subject) which cures, ameliorates or lessens the symptoms of a disease or removes (or lessens the impact of) its cause(s) (for example, the reduction in accumulation of pathological levels of lysosomal enzymes). In this case, the term is used synonymously with the term "therapy".

Additionally, the terms "treatment" or "treating" refers to an intervention (e.g. the administration of an agent to a subject) which prevents or delays the onset or progression of a disease or reduces (or eradicates) its incidence within a treated population. In this case, the term treatment is used synonymously with the term "prophylaxis".

As used herein, an *effective amount* or a *therapeutically effective amount* of an agent defines an amount that can be administered to a subject without excessive toxicity, irritation, allergic response, or other problem or complication, commensurate with a reasonable benefit/risk ratio, but one that is sufficient to provide the desired effect, e.g. the treatment or prophylaxis manifested by a permanent or temporary improvement in the subject's condition. The amount will vary from subject to subject, depending on the age and general condition of the individual, mode of administration and other factors. Thus, while it is not possible to specify an exact effective amount, those skilled in the art will be able to determine an appropriate "effective" amount in any individual case using routine experimentation and background general knowledge. A therapeutic result in this context includes eradication or lessening of symptoms, reduced pain or discomfort, prolonged survival, improved mobility and other markers of clinical improvement. A therapeutic result need not be a complete cure.

In the context of treatment and effective amounts as defined above, the term *subject* (which is to be read to include "individual", "animal", "patient" or "mammal" where context permits) defines any subject, particularly a mammalian subject, for whom treatment is indicated. Mammalian subjects include, but are not limited to, humans, domestic animals, farm animals, zoo animals, sport animals, pet animals such as dogs, cats, guinea pigs, rabbits, rats, mice, horses, cattle, cows; primates such as apes, monkeys, orangutans, and chimpanzees; canids such as dogs and wolves; felids such as cats, lions, and tigers; equids such as horses, donkeys, and zebras; food animals such as cows, pigs, and sheep; ungulates such as deer and giraffes; and rodents such as mice, rats, hamsters and guinea pigs. In preferred embodiments, the subject is a human.

### Strains and Species

*"Bifidobacterium longum* ssp *suis* APC1461 strain" refers to the strain of bacteria deposited with the National Collection of Industrial and Marine Bacteria (Ferguson Building, Craibstone Estate, Bucksburn, Aberdeen AB219YA, UK) under the Accession No. NCIMB 42832 on 29 September 2017. Characteristics of the strain are provided in the Tables below and the accompanying figures.

*"Bifidobacterium longum* ssp *longum* APC1473 strain" refers to the strain of bacteria deposited with the National Collection of Industrial and Marine Bacteria (Ferguson Building, Craibstone Estate, Bucksburn, Aberdeen AB219YA, UK) under the Accession No. NCIMB 42824 on 29 September 2017. Characteristics of the strain are provided in the Tables below and the accompanying figures.

*"Bifidobacterium longum* ssp *longum* APC1476 strain" refers to the strain of bacteria deposited with the National Collection of Industrial and Marine Bacteria (Ferguson Building, Craibstone Estate, Bucksburn, Aberdeen AB219YA, UK) under the Accession No. NCIMB 42825 on 29 September 2017. Characteristics of the strain are provided in the Tables below and the accompanying figures.

*"Bifidobacterium longum* ssp *longum* APC1478 strain" refers to the strain of bacteria deposited with the National Collection of Industrial and Marine Bacteria (Ferguson Building, Craibstone Estate, Bucksburn, Aberdeen AB219YA, UK) under the Accession No. NCIMB 42826 on 29 September 2017. Characteristics of the strain are provided in the Tables below and the accompanying figures.

*"Bifidobacterium longum* ssp *longum* APC1480 strain" refers to the strain of bacteria deposited with the National Collection of Industrial and Marine Bacteria (Ferguson Building, Craibstone Estate, Bucksburn, Aberdeen AB219YA, UK) under the Accession No. NCIMB 42827 on 29 September 2017. Characteristics of the strain are provided in the Tables below and the accompanying figures.

*"Bifidobacterium longum* ssp *longum* APC1503 strain" refers to the strain of bacteria deposited with the National Collection of Industrial and Marine Bacteria (Ferguson Building, Craibstone Estate, Bucksburn, Aberdeen AB219YA, UK) under the Accession No. NCIMB 42828 on 29 September 2017. Characteristics of the strain are provided in the Tables below and the accompanying figures.

*"Bifidobacterium longum* ssp *longum* APC289 strain" or "DPC 6317" refers to the strain of bacteria deposited with the National Collection of Industrial and Marine Bacteria (Ferguson Building, Craibstone Estate, Bucksburn, Aberdeen AB219YA, UK) under the Accession No. NCIMB 42829 on 29 September 2017. Characteristics of the strain are provided in the Tables below and the accompanying figures.

*"Bifidobacterium longum* ssp *longum* APC293 strain" or "DPC 6321" refers to the strain of bacteria deposited with the National Collection of Industrial and Marine Bacteria (Ferguson Building, Craibstone Estate, Bucksburn, Aberdeen AB219YA, UK) under the Accession No. NCIMB 42830 on 29 September 2017. Characteristics of the strain are provided in the Tables below and the accompanying figures.

*"Bifidobacterium longum* ssp *longum* APC295 strain" or "DPC 6362" refers to the strain of bacteria deposited with the National Collection of Industrial and Marine Bacteria (Ferguson Building, Craibstone Estate, Bucksburn, Aberdeen AB219YA, UK) under the Accession No. NCIMB 42831 on 29 September 2017. Characteristics of the strain are provided in the Tables below and the accompanying figures.

*"Bifidobacterium longum* ssp *longum* APC1462 strain" refers to the strain of bacteria deposited with the National Collection of Industrial and Marine Bacteria (Ferguson Building, Craibstone Estate, Bucksburn, Aberdeen AB219YA, UK) under the Accession No. NCIMB 42833 on 29 September 2017. Characteristics of the strain are provided in the Tables below and the accompanying figures.

*"Bifidobacterium longum* ssp *longum* APC1465 strain" refers to the strain of bacteria deposited with the National Collection of Industrial and Marine Bacteria (Ferguson Building, Craibstone Estate, Bucksburn, Aberdeen AB219YA, UK) under the Accession No. NCIMB 42834 on 29 September 2017. Characteristics of the strain are provided in the Tables below and the accompanying figures.

*"Bifidobacterium longum* ssp *longum* APC1464 strain" refers to the strain of bacteria deposited with the National Collection of Industrial and Marine Bacteria (Ferguson Building, Craibstone Estate, Bucksburn, Aberdeen AB219YA, UK) under the Accession No. NCIMB 42839 on 5 October 2017. Characteristics of the strain are provided in the Tables below and the accompanying figures.

*"Bifidobacterium longum* ssp *longum* APC1468 strain" refers to the strain of bacteria deposited with the National Collection of Industrial and Marine Bacteria (Ferguson Building, Craibstone Estate, Bucksburn, Aberdeen AB219YA, UK) under the Accession No. NCIMB 42840 on 5 October 2017. Characteristics of the strain are provided in the Tables below and the accompanying figures.

*"Bifidobacterium longum* ssp *longum* APC1504 strain" refers to the strain of bacteria deposited with the National Collection of Industrial and Marine Bacteria (Ferguson Building, Craibstone Estate, Bucksburn, Aberdeen AB219YA, UK) under the Accession No. NCIMB 42841 on 5 October 2017. Characteristics of the strain are provided in the Tables below and the accompanying figures.

*"Bifidobacterium longum* APC 1472" refers to the strain of bacteria deposited with the National Collection of Industrial and Marine Bacteria (Ferguson Building, Craibstone Estate, Bucksburn, Aberdeen AB219YA, UK) under the Accession No. NCIMB 42795 on 1

August 2017. Characteristics of the strain are provided in the Tables below and the accompanying figures.

*"Bifidobacterium longum* APC 1466" refers to a strain of *Bifidobacterium longum* spp. *longum* obtained from the neonatal gut having the characteristics provided in Table 1 and the Figures, especially Figures 4A and 5A. It is available from the APC Microbiome Institute (APC), UCC, Cork, Ireland under the reference APC 1466.

*"Bifidobacterium longum* APC 1477" refers to a strain of *Bifidobacterium longum* spp. *longum* obtained from the neonatal gut having the characteristics provided in Table 1 and the Figures, especially Figures 4A and 5A. It is available from the APC Microbiome Institute (APC), UCC, Cork, Ireland under the reference APC 1477.

*"Bifidobacterium longum* DPC 6316" refers to a strain of *Bifidobacterium longum* spp. *longum* obtained from the neonatal gut having the characteristics provided in Table 1 and the Figures, especially Figures 4A and 5A. It is available from the APC Microbiome Institute (APC), UCC, Cork, Ireland under the reference DPC 6316.

*"Bifidobacterium longum* DPC 6320" refers to a strain of *Bifidobacterium longum* spp. *longum* obtained from the neonatal gut having the characteristics provided in Table 1 and the Figures, especially Figures 4A and 5A. It is available from the APC Microbiome Institute (APC), UCC, Cork, Ireland under the reference DPC 6320.

Annoted sequences for strains APC 1466, 1477, DPC 6316 and 6320 are available from Genebank at the following address:
https://www.ncbi.nlm.nih.gov/nuccore?term=(((bifidobacterium%20longum)%20AND%20ar boleya%5BAuthor%5D))%20AND%20APC%201466.

Deposits of the deposited strains were made by Teagasc Food Research Centre, a Centre forming part of Agriculture and Food and Development Authority (Teagacs), on 29 September 2017 and 5 October 2017. All of the above strains are characterised to species level by 16s rRNA-internally transcribed spacer (ITS) gene sequence analysis and to strain level by pulsed field gel electrophoresis (PFGE). The strains were all isolated from faecal samples from healthy, human infants. All strains were culturable. The strains were confirmed as unique due to distinct PFGE profiles and varying phenotypic traits,

The strain may be in a viable or non-viable form, or mixtures of viable and non-viable bacteria. The strain may be provided in any format, for example as a liquid culture (or supernatant derived from a liquid culture), or in a dried or freeze-dried format. The invention may also employ growth media in which the strain of the invention was grown, or cell lysates generated using the strain of the invention. The invention also includes mutants and variants of the deposited strain that are substantially identical, genetically and phenotypically, to the deposited strain and retain the activity of the deposited strain. Thus, the invention includes derivatives of the strain that have been genetically engineered to modify the genome of the strain, typically without fundamentally altering the functionality of the organism, for example engineered for heterologous expression of a nucleic acid, or engineered to overexpress an endogenous gene, or engineered to silence a gene, to produce a recombinant or transformed strain of the invention. Genetic modifications may be carried out using recombinant DNA techniques and reagents (see below). The term also includes variants of the strain having natural or spontaneous genetic alterations. The term is also intended to encompass variant strains obtained by serial passage of the isolated strain of the invention. The variant generally has a 16S rRNA amplicon (fragment) sequence that is identical or substantially identical with the deposited strain, for example at least 99.1%, 99.2%, 99.3%, 99.4%, 99.5%, 99.6%, 99.7%, 99.8% or 99.9% identical with the deposited strain. Sequence homology can be determined using an online homology algorithm "BLAST", publicly available at http://www.ncbi.nlm.nih.gov/BLAST/.

In this specification, the term "isolated" as applied to a strain of bacteria means that the strain is isolated from other strains of bacteria. The individual isolated strains are then combined together to form the cohort of isolated bacteria comprising unique isolated strains.

In this specification, the term "unique" as applied to the strains making up the cohort of bacteria means that each strain in the cohort has a distinct PFGE profile. Methods for determining the PFGE profile of a strain of bacteria are described below.

In this specification, the term "cohort of isolated strains" refers to at least five strains of the same species, and ideally of the same sub-species. In one embodiment, the term refers to at least 6, 7, 8, 9, 10 strains of the same species.

In this specification, the term "pangenome" as applied to a specific species of bacteria means the total gene repertoire determined across a representative number of unique strains of the specific species of bacteria that are represented in a specific microbiome, for example a human microbiome or a bovine microbiome. The pangenome is therefore species specific and microbiome specific; the *Bifidobacterium longum* pangenome described below is a specific to the human gut microbiome, and more specifically the infant human gut microbiome. The number of unique strains required to compile a species-specific and microbiome-specific pangenome depends on the species concerned, and will be known to a person skilled in the art, and in the case of some species of bacteria 10-15 unique strains may be sufficient and in the case of other species (*Bifidobacterium longum* pangenome for infant human gut microbiome) more than 20 unique strains are considered to be required to provide an adequate and representative pangenome. Methods of computation of the pangenome of a bacterial species are described below, and are exemplified herein with respect to the pangenome of *Bifidobacterium longum* (*and also described in* Arboleya et al BMC Genomics 2018: 19;33*)* . Pangenomes for other species of bacteria are described in the literature, for example Lactobacilli (O'Toole et al. Nature Communications (2015) 6:8322; DOI: 10.1038), Akkermansia (Guo et al. BMC Genomics (2017) 18:800), Rhodococcus equi (Anastasi et al (2016) Genome Biol. Evol. 8(10):3140-3148, and Bifidobacterium longum (O'Callaghan et al. BMC Genomics (2015) 16:832).

In this specification, the term "representative of" as applied to a microbiome-specific pangenome of a species of bacteria means a cohort of strains of the species of bacteria that together contain at least 60% of the total gene repertoire of the pangenome of that species as determined using the methods described herein. In one embodiment, the term means a cohort of strains of the species that together contain at least 70%, 80% or 90% of the total gene repertoire of the pangenome.

In this specification, the term "capable of survival in a simulated intestinal environment" as applied to a strain of bacteria should be understood to mean that the strain is resistant to low pH, resistant to bile, and/or capable of adhering to intestinal epithelial cells as defined in the Guidelines for the Evaluation of Probiotics in Food (FAO/WHO Working Group Report April 30 and May 1 2002).

In this specification, the term "variation in sugar utilisation profiles" as applied to the cohort of isolated strains means that the cohort of strains is together capable of utilising a number of different sugars including human milk oligosaccharides and preferably plant derived carbohydrates. In one embodiment, the cohort of strains is together capable of utilising 3, 4 or all of xylo-oligosaccharides, arabinan, arabinoxylan, galactan, and fucosyllactose.

In this specification, the term "intestinal origin" as applied to a strain of bacteria means that the bacteria is derived from the intestine of a mammal.

### Compositions and Foods

The invention also relates to a composition comprising a cohort of strains according to the invention, or a composition comprising one or more strains of the invention. The composition may be a pharmaceutical composition, or a food composition, or a dietary supplement composition. The term "food" refers to a man-made food product including beverages, food additives and food supplements. Examples of foods include dairy products such as milk, yoghurt, cheese, cheese food, dairy powders, probiotic formulations, infant formula powders, follow-on milk formula, food for special medicinal purposes, meat products, soups, vegetable products, fruit juices, fruit products, breads, confectionary, cakes, sports supplements, nutritional supplements and the like.

In one embodiment, the composition includes a probiotic material. In one embodiment, the composition comprises a prebiotic material.

"Probiotic" refers to a microbial cell preparation, typically a bacterial cell preparation, that exerts a beneficial effect on the health or well-being of a host. They are described in Salminen et al (Trends Food Sci. Technol. 1999:10 107-110).

"Prebiotic" refers to a material or composition that can promote the growth of probiotic microbes or bacteria, especially bacterial growth in the mammalian gastrointestinal tract. Examples include oligosaccharides, dietary fibres, or mixtures thereof. Exemplary prebiotics are described in WO2011/039176, pages 12 to 15.

The invention also relates to pharmaceutical compositions which comprise pharmaceutical carriers.

As used herein, the term "pharmaceutical composition" refers to a therapeutically effective amount of the strain of the invention, and a pharmaceutically acceptable carrier. In a specific embodiment, the term "pharmaceutically acceptable" means approved by a regulatory agency of the Federal or a state government or listed in the U.S. Pharmacopeia or other generally recognized pharmacopeia for use in animals, and more particularly in humans. In the case of the present invention, the term "therapeutically effective amount" should be taken to mean an amount of therapeutic which results in a clinically significant increase in proliferation of target cells, for example gut epithelial cells or skin epithelial cells.

As used herein, the term "adjuvant" means an agent that enhances the recipient's immune response to an immunogenic peptide or protein. Details of suitable adjuvant compositions are well known to those skilled in the art.

As used herein, the term "pharmaceutically acceptable carrier" refers to a diluent, adjuvant, excipient, or vehicle with which the Therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene glycol, water, ethanol and the like.

The composition, if desired, can also contain minor amounts of wetting or emulsifying agents, or pH buffering agents. These compositions can take the form of solutions, suspensions, emulsion, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E. W. Martin. Such compositions will contain a therapeutically effective amount of the therapeutic, preferably in purified form, together with a suitable amount of carrier so as to provide the form for proper administration to the patient. The formulation should suit the mode of administration.

In a preferred embodiment, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lignocaine to, ease pain at the, site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

Pharmaceutical compositions formulated or configured for oral administration and gastric transit are known in the art and include those described below:
- Sathish et al (Int. J. Pharm. Sci.2013; 258-269);
- Kushal et al (Int. Res. J. Pharm. 2013, 4(3));
- Philip et al (Oman Med J. 2010, 25(2));
- Polymers for controlled drug delivery - Peter Tarcha (CRC Press, 21 November 1990);
- Pharmaceutical coating technology - Michael Aulton et al (Taylor & Francis 27 October 1995);
- http://www.slideshare.net/Balimusale/oral-controlled-drug-delivery-system;
- European Patent No: 2418968 (Teagasc); and
- European Patent No: 2097072 (RCSI).
- Brayden et al. (European Journal of Pharmaceutical Sciences 79 (2015), 102-111.
- Tambuwala et al. (Journal of Controlled Release 217 (2015) 221-227.
- Zhang et al. Evaluation of alginate-whey protein microcapsules for intestinal delivery of lipophilic compounds in pigs (J. Sci. Food Agric. (2015).
- Lamprecht et al. (Journal of Controlled Release 104 (2005) 337-346.
- Hua et al. (Nanomedicine: Nanotechnology, Biology and Medicine 11 (2015) 1117-1132.
- Drug Delivery: Fundamentals and Applications (Chapter 7, Oral Drug Delivery, Hillary and Brayden)

As used herein, the term "food" refers to a man-made food product including beverages, food additives and food supplements. Examples of foods include dairy products such as milk, yoghurt, cheese, cheese food, dairy powders, probiotic formulations, infant formula powders, follow-on milk formula, food for special medicinal purposes, meat products, soups, vegetable products, fruit juices, fruit products, breads, confectionary, cakes, sports supplements, nutritional supplements and the like.

### Dosage

It is preferable that the strain or composition is administered at least once per week over a treatment period of at least 4 weeks, and preferably at least 5, 6, 7, 8, 9, 10, 11, 12, 14, 16, 18 or 20 week period. Preferably, the strain or composition is administered several times a week, and ideally once a day. Compositions of the invention generally comprise between 10³ and 10¹² cfu of the strain of the invention per gram of dry weight of the composition. In one embodiment, the composition comprises10³ and 10¹² cfu, or 10⁴ and 10¹² cfu, or 10⁶ and 10¹⁰ cfu of the strain of the invention per gram of dry weight of the composition. A daily dose generally comprises between 10³ and 10¹² cfu of the strain. In one embodiment, the daily dose comprises10³ and 10¹² cfu, or 10⁴ and 10¹² cfu, or 10⁶ and 10¹⁰ cfu of the strain.

### Recombinant DNA techniques and reagents

In one aspect, the invention relates to isolated *Bifidobacterium longum* strains provided above. The invention also relates to mutants and variants of the strain that are substantially identical to the deposited strain and exhibit the same weight modification functionality. This includes strains that are genetically engineered to alter the genome of the deposited strain (i.e. strained engineered for heterologous expression of nucleic acid), and variants obtained through natural genetic alterations such as spontaneous mutations, adaption and serial passage the term "engineered" as applied to a cell means genetically engineered using recombinant DNA technology, and generally involves the step of synthesis of a suitable expression vector (see below) and then transfecting (i.e. stably or transiently) the expression vector into a host cell (generally stable transfection). The term "heterologous expression" refers to expression of a nucleic acid in a host cell that does not naturally have the nucleic acid. Insertion of the nucleic acid into the heterologous host is performed by recombinant DNA technology. The term "heterologous in-situ expression" as applied to a bacterium of the invention means that the bacterium is capable of expressing the nucleic acid in-situ in the mammalian gut, especially in-situ expression when adhered to the epithelial layer of the gut. As used herein, the term "recombinant bacterium" or "transformed bacterium" refers to a bacterium comprising an exogenous nucleic acid stably integrated into the cellular genome. In another embodiment, the present invention provides a cell comprising a non-integrated (i.e., episomal) exogenous nucleic acid, such as a plasmid, cosmid, phagemid, or linear expression element, which comprises a sequence coding suitable for expression of an exogenous nucleic acid. In other embodiments, the present invention provides a cell line produced by stably transfecting a host cell with a plasmid comprising an expression vector of the invention.

As used herein, the term "expression vector" may be any suitable vector, including chromosomal, non-chromosomal, and synthetic nucleic acid vectors (a nucleic acid sequence comprising a suitable set of expression control elements) suitable for heterologous expression of a nucleic acid. Examples of such vectors include derivatives of SV40, bacterial plasmids, phage DNA, baculovirus, yeast plasmids, vectors derived from combinations of plasmids and phage DNA, and viral nucleic acid (RNA or DNA) vectors. In one embodiment, the Tad pilus encoding nucleic acid molecule is comprised in a naked DNA or RNA vector, including, for example, a linear expression element (as described in, for instance, Sykes and Johnston, Nat Biotech 12, 355-59 (1997)), a compacted nucleic acid vector (as described in for instance U.S. Pat. No. 6,077,835 and/or WO 00/70087), or a plasmid vector such as pBR322, pUC 19/18, or pUC 118/119. Such nucleic acid vectors and the usage thereof are well known in the art (see, for instance, U.S. Pat. No. 5,589,466 and U.S. Pat. No. 5,973,972). In one embodiment, the DNA comprises an expression control sequence.

In one embodiment, the vector is suitable for heterologous expression of a nucleic acid in a bacterial cell. Examples of such vectors include expression vectors such as BlueScript (Stratagene), pIN vectors (Van Heeke & Schuster, 1989, J Biol Chem 264, 5503-5509), pET vectors (Novagen, Madison, Wis.) and the like. In one embodiment, the expression vector may also or alternatively be a vector suitable for expression in a yeast system. Any vector suitable for expression in a yeast system may be employed. Suitable vectors include, for example, vectors comprising constitutive or inducible promoters such as yeast alpha factor, alcohol oxidase and PGH (reviewed in: F. Ausubel et al., ed., 1987, Current Protocols in Molecular Biology, Greene Publishing and Wiley InterScience New York; and Grant et al., 1987, Methods in Enzymol 153, 516-544). In other embodiments, the expression vector is suitable for expression in baculovirus-infected insect cells. (Kost, T; and Condreay, J P, 1999, Current Opinion in Biotechnology 10 (5): 428-33.)

Expression control sequences are engineered to control and drive the transcription of genes of interest, and subsequent expression of proteins in various cell systems. Plasmids combine an expressible gene of interest with expression control sequences (i.e. expression cassettes) that comprise desirable elements such as, for example, promoters, enhancers, selectable markers, operators, etc. In an expression vector of the invention, Tad pilus-encoding nucleic acid molecules may comprise or be associated with any suitable promoter, enhancer, selectable marker, operator, repressor protein, polyA termination sequences and other expression-facilitating elements.

"Promoter" as used herein indicates a DNA sequence sufficient to direct transcription of a DNA sequence to which it is operably linked, i.e., linked in such a way as to permit transcription of the exogenous nucleotide sequence when the appropriate signals are present. The expression of a nucleotide sequence may be placed under control of any promoter or enhancer element known in the art. Examples of such elements include strong expression promoters (e.g., human CMV IE promoter/enhancer or CMV major IE (CMV-MIE) promoter, as well as RSV, SV40 late promoter, SL3-3, MMTV, ubiquitin (Ubi), ubiquitin C (UbC), and HIV LTR promoters). In some embodiments, the vector comprises a promoter selected from the group consisting of SV40, CMV, CMV-IE, CMV-MIE, RSV, SL3-3, MMTV, Ubi, UbC and HIV LTR.

Nucleic acid molecules of the invention may also be operably linked to an effective poly (A) termination sequence, an origin of replication for plasmid product in E. coli, an antibiotic resistance gene as selectable marker, and/or a convenient cloning site (e.g., a polylinker). Nucleic acids may also comprise a regulatable inducible promoter (inducible, repressable, developmentally regulated) as opposed to a constitutive promoter such as CMV IE (the skilled artisan will recognize that such terms are actually descriptors of a degree of gene expression under certain conditions).

Selectable markers are elements well-known in the art. Under the selective conditions, only cells that express the appropriate selectable marker can survive. Commonly, selectable marker genes express proteins, usually enzymes, that confer resistance to various antibiotics in cell culture. In other selective conditions, cells that express a fluorescent protein marker are made visible, and are thus selectable. Embodiments include beta-lactamase (bla) (beta-lactam antibiotic resistance or ampicillin resistance gene or ampR), bls (blasticidin resistance acetyl transferase gene), bsd (blasticidin-S deaminase resistance gene), bsr (blasticidin-S resistance gene), Sh ble (Zeocin^{®} resistance gene), hygromycin phosphotransferase (hpt) (hygromycin resistance gene), tetM (tetracycline resistance gene or tetR), neomycin phosphotransferase II (npt) (neomycin resistance gene or neoR), kanR (kanamycin resistance gene), and pac (puromycin resistance gene).

In certain embodiments, the vector comprises one or more selectable marker genes selected from the group consisting of bla, bls, BSD, bsr, Sh ble, hpt, tetR, tetM, npt, kanR and pac. In other embodiments, the vector comprises one or more selectable marker genes encoding green fluorescent protein (GFP), enhanced green fluorescent protein (eGFP), cyano fluorescent protein (CFP), enhanced cyano fluorescent protein (eCFP), or yellow fluorescent protein (YFP).

For the purposes of this invention, gene expression in eukaryotic cells may be tightly regulated using a strong promoter that is controlled by an operator that is in turn regulated by a regulatory protein, which may be a recombinant "regulatory fusion protein" (RFP). The RFP consists essentially of a transcription blocking domain, and a ligand-binding domain that regulates its activity. Examples of such expression systems are described in US20090162901A1.

As used herein "operator" indicates a DNA sequence that is introduced in or near a gene in such a way that the gene may be regulated by the binding of the RFP to the operator and, as a result, prevents or allow transcription of the gene of interest, i.e. a nucleotide encoding a polypeptide of the invention. A number of operators in prokaryotic cells and bacteriophage have been well characterized (Neidhardt, ed., Escherichia coli and Salmonella; Cellular and Molecular Biology 2d. Vol 2 ASM Press, Washington D.C. 1996). These include, but are not limited to, the operator region of the LexA gene of E. coli, which binds the LexA peptide, and the lactose and tryptophan operators, which bind the repressor proteins encoded by the Lad and trpR genes of E. coli. These also include the bacteriophage operators from the lambda PR and the phage P22 ant/mnt genes, which bind the repressor proteins encoded by lambda cl and P22 arc. In some embodiments, when the transcription blocking domain of the RFP is a restriction enzyme, such as Notl, the operator is the recognition sequence for that enzyme. One skilled in the art will recognize that the operator must be located adjacent to, or 3' to the promoter such that it is capable of controlling transcription by the promoter. For example, U.S. Pat. No. 5,972,650 specifies that tetO sequences be within a specific distance from the TATA box. In specific embodiments, the operator is preferably placed immediately downstream of the promoter. In other embodiments, the operator is placed within 10 base pairs of the promoter.

In an exemplary cell expression system, cells are engineered to express the tetracycline repressor protein (TetR) and a protein of interest is placed under transcriptional control of a promoter whose activity is regulated by TetR. Two tandem TetR operators (tetO) are placed immediately downstream of a CMV-MIE promoter/enhancer in the vector. Transcription of the gene encoding the protein of interest directed by the CMV-MIE promoter in such vector may be blocked by TetR in the absence of tetracycline or some other suitable inducer (e.g. doxycycline). In the presence of an inducer, TetR protein is incapable of binding tetO, hence transcription then translation (expression) of the protein of interest occurs. (See, e.g., U.S. Pat. No. 7,435,553.)

The vectors of the invention may also employ Cre-lox recombination tools to facilitate the integration of a gene of interest into a host genome. A Cre-lox strategy requires at least two components: 1) Cre recombinase, an enzyme that catalyzes recombination between two IoxP sites; and 2) IoxP sites (e.g. a specific 34-base pair by sequence consisting of an 8-bp core sequence, where recombination takes place, and two flanking 13-bp inverted repeats) or mutant lox sites. (See, e.g. Araki et al., 1995, PNAS 92:160-4; Nagy, A. et al., 2000, Genesis 26:99-109; Araki et al., 2002, Nuc Acids Res 30(19):e103; and US20100291626A1). In another recombination strategy, yeast-derived FLP recombinase may be utilized with the consensus sequence FRT (see also, e.g. Dymecki, S. M., 1996, PNAS 93(12): 6191-6196).

### Exemplification

The invention will now be described with reference to specific Examples. These are merely exemplary and for illustrative purposes only: they are not intended to be limiting in any way to the scope of the monopoly claimed or to the invention described. These examples constitute the best mode currently contemplated for practicing the invention.

### METHODS

### Bacterial strains and growth conditions

The twenty Bifidobacterium longum strains used(Table 3) had previously been isolated from infant faeces, except in two cases where the strains originate from adult faeces, and deposited in the APC Culture Collection (APC Microbiome Institute, Ireland). Strains coded as DPC were isolated by Barrett and coworkers (Barrett E, Ross RP, Fitzgerald GF, & Stanton C (2007) Rapid screening method for analyzing the conjugated linoleic acid production capabilities of bacterial cultures. Applied and environmental microbiology 73(7):2333-2337.). Bifidobacteria were routinely grown on de Man-Rogose-Sharpe (MRS) medium (Difco Laboratories, Detroit, MI) supplemented with 0.05% w/v cysteine-HCl (Sigma, St. Louis, MO) (MRSC) and incubated at 37°C under anaerobic conditions (10% H2, 10% CO2, and 80% N2) in a chamber Mac 500 (Don Whitley Scientific, West Yorkshire, UK). For solid medium, 2% (w/v) agar (Oxoid, Basingstoke, UK) was added. Prior to each DNA extraction bacteria were sub-cultured twice and overnight cultures were used.

### Genomic DNA extraction, sequencing and data assembly

DNA was isolated by using DNeasy Blood & Tissue Kit (Qiagen, Sussex, UK) following the manufacturer's instructions. Briefly, the overnight culture were centrifuged, washed with PBS (Sigma, St. Louis, MO) and incubated for 1 hour at 37oC in an enzymatic lysis buffer with lysozyme (50mg/ml) (Sigma, St. Louis, MO). Then, manufacturer's protocol was followed with the extra addition of RNAse (Sigma, St. Louis, MO). Sequencing and assembly was performed by Eurofins Genetic Services Ltd. (Ebersberg, Germany). The genomic DNA was sequenced on an Illumina MiSeq platform using chemistry v3 with paired-end sequencing. The draft genomes were de novo assembled following a pipeline that incorporates Velvet software (v1.2.10) (50) and a multi-kmer approach (51).

### Contig analysis and general feature prediction

A preliminary comparative genomic analysis (methods explained in the next section) was conducted to align the twenty *B. longum* APC/DPC genomes with all complete genomes of *B. longum* available in the NCBI public database at the moment of the study (Table 5), in order to select the most appropriate genome of reference for ordering the contigs prior to annotation. The alignment of the twenty *B. longum* draft genome sequences was performed against a number of closely related, fully sequenced, currently and publicly available *B. longum* genomes (Table 5). The *B. longum* JDM301, *B. longum* BBMN68, B. *longum* NCIMB 8809, *B. longum* DJ010A and *B. longum* GT15 genomes were thus selected as reference genomes so as to determine presumed contig order and orientation of each draft genome. MAUVE (v2.3.1) was used to reorder contigs based on the reference genome along with Artemis software (v.14) for visualization and manual editing of the beginning of each genome at the dnaA gene. Prediction of putative open reading frames (ORFs) was carried out using Prodigal predictor v2.50 software (http://prodigal.ornl.gov). Identified ORFs were then automatically annotated on the basis of BLASTP analysis (Altschul SF, Gish W, Miller W, Myers EW, & Lipman DJ (1990) Basic local alignment search tool. Journal of molecular biology 215(3):403-410.). Functional assignment was performed against the non-redundant protein data base provided by the National Centre for Biotechnology (https://www.ncbi.nlm.nih.gov). Artemis was also used for inspecting and editing, where necessary, the ORF finding outputs and the associated BLASTP results. Moreover, the annotations were further refined and verified using the protein family (Pfam) (http://pfam.xfam.org) database. Glycosyl hydrolases were predicted and annotated based on similarity to the Carbohydrate-Active enZYmes (CAZy) (http://www.cazy.org/) database, Enzyme Commission number (EC) database (http://enzyme.expasy.org) using annot8r pipeline (http://www.nematodes.org/bioinformatics/annot8r) and Pfam alignments

### Comparative genomics

An all-versus-all BLASTP alignment (Altschul et al.) (50% identity; e-value 1e-4 cut-off) was performed on the extracted protein sequences from each strain. The BLAST output were used as an input for the clustering into protein families sharing the same function using the Markov Cluster Algorithm (MCL) with an inflation index of 2.5, as previously described (Bottacini F, *et al.* (2014) Comparative genomics of the Bifidobacterium breve taxon. BMC genomics 15:170.). The obtained gene families were classified as belonging to either the core or to the variable genome based on their presence in either all strains or in a subset of the investigated strains, respectively. In the orthologue extraction an additional filter for paralogues was applied by selecting only those families that were shown to contain a single protein member for each genome.

### Pangenome analyses

In order to predict the possible dynamic changes of genome size at the genus, the sizes of pangenome, core genome and unique gene were computed. For all the genomes used in this study, a pangenome calculation was done using PGAP (Zhao Y, et al. (2012) PGAP: pan-genomes analysis pipeline. Bioinformatics (Oxford, England) 28(3):416-418.), as previously described (Bottacini et al.). The ORF content of each genome was organized in functional gene clusters using the gene family (GF) method implemented in the PGAP pipeline. A pangenome profile and a core genome profile were built using all possible BLAST combinations for each genome being sequentially added.

### Phylogenetic analyses

The supertree computation was performed from the alignment of a set of orthologous genes obtained from a BLAST-based comparative approach as indicated above, with our APC/DPC strains plus all the *B. longum* genomes available in the NCBI public database at the time of writing. Each set of orthologous proteins was aligned using MUSCLE (Edgar RC (2004) MUSCLE: a multiple sequence alignment method with reduced time and space complexity. BMC bioinformatics 5:113.) and phylogenetic trees were constructed using the maximum-likelihood in PhyML (Guindon S, *et al.* (2010) New algorithms and methods to estimate maximum-likelihood phylogenies: assessing the performance of PhyML 3.0.

Systematic biology 59(3):307-321.). The resulting consensus tree was computed using the Consense module from Phylip package v3.69 using the majority rule method (http://evolution.genetics.washington.edu/phylip.html) and phylogenetic data were submitted to TreeBASE database (http://treebase.org/treebase-web/home.html).

### Bifidobacterial growth on different carbohydrate sources and GTM associations

For evaluation of growth on the different carbohydrates sources, strains were cultured on modified MRS (mMRS) medium manually prepared with the following composition: tryptone (10.0 g/L), yeast extract (5 g/L), beef extract (10.0 g/L), K2HPO4 (3.0 g/L), KH2PO4 (3.0 g/L), tri-ammonium citrate (2.0 g/L), pyruvic acid (0.2 g/L), cysteine-HCl (0.3 g/L), Tween-80 (1 mL), MgSO4·7H2O (0.575 g/L), MnSO4·4H2O (0.12 g/L), FeSO4·7H2O (0.034 g/L). Prior to autoclaving, mMRS medium was adjusted to pH 6.8.

Thirty-one carbohydrates were tested in this study (Table7). Solutions of most of them were prepared by dissolution at 5% (w/v) in distilled water and sterilized by filtration (0.45 µm). They were then added to mMRS medium at final concentration of 0.5% (v/v). Since certain carbohydrates do not easily dissolve in water (pullulan, starch, amylopectin, inulin, arabinoxylan (wheat and rye), pectin galactan, mucin, galactan potato, pectin apple, arabinan, arabinogalactan, xylan and amylopectin), they were added directly to mMRS medium at a final concentration of 0.5% (w/v) and autoclaved at 121°C for 15 min.

For growth assays Bifidobacterium strains were cultured at 37°C under anaerobic conditions in 10 ml of MRSC. Afterwards they were sub-cultured twice, first at 2% (v/v) into 10 mL of fresh medium during 8 hours, and then at 1% (v/v) into 10 mL of MRSC fresh medium overnight. The next day, strains were inoculated at 1% (v/v) into 10 mL of mMRS medium, previously supplemented with the soluble carbohydrates at a final concentration of 0.5% (v/v) or directly to mMRS with non-soluble carbohydrate autoclaved. Prior to inoculation both media were supplemented with cysteine-HCl at final concentration of 0.05%.

Growth of the bacterial strains was evaluated by optical density (OD600 nm) using UV-1280 spectrophotometer (Schimadzu Corporation, Kyoto, Japan). Measurements were taken manually over a 24-hour period at different time points: 0, 6, 9, 12 and 24 hours. mMRS supplemented with 0.05% w/v cysteine-HCl and without the addition of a carbohydrate source served as a negative control. Based on the OD600 nm values at 12 hours, bacterial growth was categorized as good growth (+) with an OD600 nm higher than 0.5 and moderate growth (+-) with an OD600 nm between 0.4 and 0.5. A cut-off value of 0.4 OD600 nm was used to discriminate strains which were not able to grow in the given carbohydrates.

Following completion of the fermentation assessment of *B. longum* strains, an in-silico genotype/phenotype gene-trait matching (GTM) exercise was performed, correlating the presence/absence of genes obtained from comparative analysis with an observed phenotype (Table 4 - Fig. 5).

The analysis was performed on a subset of gene families obtained after exclusion of the ones present in all the strains (core-genome). A further filter was included which excluded from the analysis that fraction of genes not involved in carbon sources utilization (e.g. transposases, R/M systems, CRISPR systems and prophages).

After obtaining the final number of families a further reduction of the dataset in clusters of unique combination of occurrence was performed which allowed to obtain the "genotype" binary matrix (values 0 for absence and 1 for presence of a gene family). This obtained matrix contained 372 clusters on the rows and 20 strains on the columns. A second binary matrix was also similarly generated containing the fermentation profile and constituting the "phenotype". In this case a value of 0 was assigned for OD600 <0.3 and value 1 for OD600 >0.4 (taken at 12 hours of growth curve). The "phenotype" binary matrix contained 16 carbohydrates on the rows and 20 strains on the columns, organized in the same order as in the genotype.

Following alignment of these two matrices the matching percentage between each row of the two matrices and the result was represented as a heatmap. The positive matches obtained from the analysis (> 95 % of match between "genotype" and "phenotype") as well as the relative genomic surrounded regions were further inspected and compared with additional information retrieved from alternative available databases such as EC (Enzyme Classification) database (Bairoch A (2000) The ENZYME database in 2000. Nucleic Acids Research 28(1):304-305.) and PFAM (http://pfam.sanger.ac.uk) alignments, in order to further support the obtained results

### Acid tolerance

Bifidobacterium strains were tested for their ability to survive under acidic conditions according to the method by Liong and Shah (2005) with some minor adjustments as follows: Fresh cultures were grown for 48 hours in 10 ml mMRS, harvested by centrifugation (2263 g × 10 min) and then washed once in an equal amount of Dulbecco's Phosphate buffered Saline (PBS) (Sigma Aldrich, Wicklow, Ireland). Bacterial cells were then immediately resuspended in mMRS, adjusted to pH 2.5 with 5 M HCL, and then incubated under anaerobic conditions at 37 °C. Aliquots were withdrawn at time point 0 and 30, 60, 90 and 120 minutes. A 10 fold dilution of each aliquot was performed in maximum recovery diluent (MRD) (Oxoid, Basingstoke, Hampshire, England) followed by pour plating onto RCM. The plates were incubated in 37 °C for 72 h under anaerobic conditions.

### Bile tolerance

To determine the ability of 29 B. longum strains to survive in the presence of 0.3 % (w/v) bovine bile (Sigma Aldrich), bacterial cells from cultures were grown for 48 h in 10 ml mMRS, harvested by centrifugation (2263 g × 10 min) and washed once in PBS and were resuspended in mMRS media containing 0.3% (w/v) bovine bile and adjusted to pH 7.0 with 4 M NaOH. Bacteria were then incubated under anaerobic conditions. Serial 10-fold dilutions were made in MRD followed by pour plating on RCM agar. Bacterial growth was monitored by viable cell counts after an incubation period of 72 hours in 37C under anaerobic conditions.

### Adhesion to HT-29 cells

### Preparation of HT-29 cells

HT-29 cells were obtained from ATCC, Virginia, USA. The cell line was cultured in McCoy's 5A medium (Sigma Aldrich) supplemented with 10 % (v/v) fetal bovine serum (FBS) (Sigma Aldrich) and 1% of antibiotic mixture (penicillin-streptomycin; Sigma Aldrich), seeded (2×105 cells / ml-1) into 12-well plates (Sigma Aldrich) and incubated for 7±1 days in 37°C, 5% CO2 until a confluent monolayer was reached (approx 2×106 cells/ml-1). One day prior to assay, the media was changed to antibiotic-free McCoy's 5A medium supplemented with 2 % FBS.

### Adhesion assay

Based on the ability of B. longum strains to tolerate various concentrations of acid and bile, the best surviving strains were tested for their ability to adhere to HT-29 cells. Prior to the adhesion assay, the confluent monolayers of HT-29 cells grown on 12-well plates were washed twice by swirling the plates with PBS, pre-warmed to 37°C. Fresh bacterial cultures, grown for 48 hours in mMRS, were harvested by centrifugation (2263 × g for 10 minutes), washed 3 times in PBS and then resuspended in 10 ml antibiotic-free and serum-free McCoy's 5A medium. Aliquots of bacterial suspension (approx. 8.5 ×108-1×109) were added to the HT-29 cells and incubated for 4 h at 37°C in 5% CO2. After 4 h, cells were rinsed 3 times in order to remove all non-adherent bacteria and were then incubated together with 0.5 ml / well of trypsin-EDTA (Sigma Aldrich) in 37°C for 15 minutes in order to release the cells. Thereafter, 1.5 ml/well of PBS was added to inactivate the trypsin. The level of adhesion was estimated by 10-fold dilution series in MRD and pour plating with RCM after incubation in 37°C for 72 h. Each strain was tested in triplicate and assay was then repeated on an independent occasion.

### Statistical analysis

Data from acid tolerance assay and adhesion assay are presented as mean ± standard error mean (SEM).

### C-section Model

C-section delivery has recently been associated with decreased colonization rates of Bifidobacterium, Bacteroides and Lactobacillus, with a decrease in diversity and richness of the microbiota. It has also been associated with an increased risk of developing obesity, type 1 diabetes, as well as immune disorders such as asthma or allergies. The Cesarian section (C-section) mouse model is a method of neonate/ infant extraction from the mothers uterus, as opposed to a natural birth, or per vaginum. At full term (Gestation day 21-22 in a mouse), the mother is euthanized by cervical dislocation. To reduce bacterial contamination of the abdominal cavity, the abdominal skin is prepped by application of isopropyl alcohol and the abdominal skin is retracted. The abdomen is incised using a clean scalpel or sharp scissors. The uterus is removed and placed on sterile gauze sponges. To prevent hypothermia of the fetus in the uterus, the gauze sponges are placed on clean tissue and a heating pad is placed beneath to provide thermal support. The individual fetus is removed by cutting the uterus gently with a sharp scissors. The pups (Gestation day 21-22) are then expelled by gentle pressure, and the umbilical cord is cut approximately 1-3 mm distal to the umbilical attachment. Cotton swabs are used to tear the amniotic membrane and massage each pup gently until spontaneous breathing is noted. If a pup does not breathe spontaneously within 30-60 s after initiating physical stimulation, a cotton swab is applied gently to the oral mucous membrane of the affected animal for 1-3 s, and physical stimulation is continued until spontaneous respiration was observed. Additional pregnant females are allowed to deliver spontaneously and the litters are used as full-term natural delivery control and as foster mothers. The pups may be dried by smearing them with the bedding material in the cage of foster mother. This model allows the comparing and contrasting of differing microbiota expression profiles between the two forms of birth (in humans) and examine negative health effects as a result of an unnatural birthing process. It allows interventional therapies to be developed to mitigate the adverse health effects from the lack of an initial per vaginum microbiota inoculation. C-section delivered babies seem to be dominated by bacteria typically present on the skin and in the environment, predominantly from non-maternal sources.

### TABLES

**Table 3. Bifidobacterium longum genomes sequenced and object of analysis in this study, w: weeks; y: years; d: days; CS: C-Section; ND: natural birth; BM: breast-milk; FM:formula milk; NA: not applicable**

| **Genomes Sub** | **Source of Isolation** | | | | **Contings** | **No. of ORFs** | **Genome Size (bp)** | **GC content** | **No. of unique genes** | **Reference** |
|---|---|---|---|---|---|---|---|---|---|---|
| | **ject** | ***Age*** | ***Delivery*** | ***Feeding*** | | | | | | |
| ***B. longum* APC 1461** | A | 27 w | CS | BM | 38 | 2020 | 2424450 | 60.0 | 60 | This study |
| ***B. longum* APC 1462** | B | 1 w | ND | BM | 28 | 2016 | 2423258 | 60.2 | 4 | This study |
| ***B. longum* APC 1464** | B | 1 w | ND | BM | 32 | 1939 | 2351990 | 60.0 | 3 | This study |
| ***B. longum* APC 1465** | C | 4 w | ND | BM | 58 | 2076 | 2457685 | 59.6 | 6 | This study |
| ***B. longum* APC 1466** | C | 27 w | ND | BM | 52 | 2057 | 2425450 | 59.8 | 0 | This study |
| ***B. longum* APC 1468** | C | 27 w | ND | BM | 46 | 2033 | 2400632 | 60.1 | 12 | This study |
| ***B. longum* APC 1472** | E | 1 w | CS | BM | 51 | 1965 | 2369515 | 60.1 | 3 | This study |
| ***B. longum* APC 1473** | F | 27 w | CS | BM | 40 | 1901 | 2322545 | 59.8 | 6 | This study |
| ***B. longum* APC 1476** | G | 27 w | ND | BM | 49 | 2180 | 2538014 | 60.0 | 1 | This study |
| ***B. longum* APC 1477** | H | 1 w | ND | BM | 25 | 1775 | 2234263 | 59.8 | 0 | This study |
| ***B. longum* APC 1478** | H | 4 w | ND | BM | 22 | 1777 | 2228826 | 59.8 | 1 | This study |
| ***B. longum* APC 1480** | J | 4 w | ND | BM | 28 | 2103 | 2483224 | 59.9 | 6 | This study |
| ***B. longum* APC 1482** | D | 1 w | ND | FM | 73 | 1955 | 2342906 | 60.1 | 17 | This study |
| ***B. longum* APC 1503** | D | 1 w | ND | FM | 40 | 2211 | 2568177 | 59.7 | 23 | This study |
| ***B. longum* APC 1504** | I | 1 w | ND | BM | 52 | 1923 | 2315762 | 60.2 | 14 | This study |
| ***B. longum* DPC 6316** | K | 25 y | NA | NA | 33 | 2014 | 2399437 | 60.4 | 23 | (49) |
| ***B. longum* DPC 6317** | L | 3 d | NA | NA | 21 | 2009 | 2454098 | 60.2 | 8 | (49) |
| ***B. longum* DPC 6320** | M | 64 y | NA | NA | 26 | 1865 | 2335839 | 59.9 | 9 | (49) |
| ***B. longum* DPC 6321** | N | 3 d | NA | NA | 29 | 1967 | 2387834 | 59.9 | 24 | (49) |
| ***B. longum* DPC 6323** | O | 4 d | NA | NA | 53 | 2016 | 2407907 | 60.2 | 7 | (49) |

**Table 4. Gene-trait matching with functions resulting from hierarchical clustering analysis.**

| **Carbohydrate** | **Gene cluster** | **Functions** |
|---|---|---|
| **XYLO-OLIGOSACCHARIDES (XOS)** | **A** | Hypothetical protein |
| | | Putative outer membrane protein |
| | | Galactoside O-acetyltransferase |
| | | Alpha-L-arabinofuranosidase |
| | | **Beta-1,4-xylosidase** |
| | | ABC transporter permease |
| | | ABC transporter permease |
| | | Lactose ABC transporter substrate-binding protein |
| | | Lacl family transcriptional regulator |
| | | NADH-dependent butanol dehydrogenase 1 |
| **ARABINAN** | **B** | **Endo-1,4-beta-xylanase** |
| | | **Endo-1,4-beta-xylanase** |
| | | **Beta-xylosidase** |
| | | **Endo-1,4-beta-xylanase** |
| **ARABINOXYLAN** | **C** | ABC transporter, permease protein, probably fructooligosaccharide porter |
| | | ABC transporter, permease protein, probably fructooligosaccharide porter |
| | | ABC transporter, extracellular SBP, probably fructooligosaccharide porter |
| | | **Exo-alpha-L-arabinofuranosidase II** |
| | | Lipase |
| | | Lacl family transcriptional regulator |
| | | **Beta-xylosidase** |
| | | **Beta-xylosidase** |
| | | **Beta-xylosidase o endo-arabinase** |
| | | **Alpha-arabinofuranosidase I** |
| **GALACTAN** | **D** | ATP-binding and permease modules of ABC transporter system |
| | | Putative transport protein |
| | | Solute-binding protein of ABC transporter system for sugars galactan |
| | | metabolism |
| | | ABC transporter permease |
| | | ABC transporter permease |
| | | Beta-galactosidase galactan metabolism |
| | | Transcriptional regulator Lacl family galactan metabolism |
| | | **Glycosyl hydrolases family 53 Endogalactanase galactan metabolism** |
| | | 25-diketo-D-gluconic acid reductase |
| **FUCOSYLLACTOSE (FL)** | **E** | Lacl family transcriptional regulator |
| | | putative ABC transporter permease |
| | | ABC transporter permease |
| | | ABC transporter substrate binding component |
| | | Mandelate racemase/muconate lactonizing protein |
| | | Short chain dehydrogenase |
| | | Hypothetical protein |
| | | Dihydrodipicolinate synthase |
| | | Predicted fucose isomerase |
| | | **Alpha-1,3/4-fucosidase Hypothetical protein (Glycosyl hydrolases family 95)** |

**Table 5. Bifidobacterium longum genomes publicly available used for different analysis along the study *At the moment of the analysis.**

| **Genomes** | **Code** | **Used in Reference Selection** | **Used in Comparative Analysis** | **Used in the ORF prediction** | **Used** | **in prediction of GHs Used in Pangenome computation** | **Status of the genome*** | **Used in Phylogenetics analysis** | **Reference** |
|---|---|---|---|---|---|---|---|---|---|
| ***B. longum* JDM301** | BLJ | x | x | x | x | x | x | complete | NCBI database |
| ***B. longum* BBMN68** | BBMN68 | x | x | x | x | x | x | complete | NCBI database |
| ***B. longum* NCIMB 8809** | B8809 | x | x | x | x | x | x | complete | NCBI database |
| ***B. longum* DJO10A** | BLD | x | x | x | x | x | x | complete | NCBI database |
| ***B. longum* GT15** | BLGT | x | x | x | x | x | x | complete | NCBI database |
| ***B. longum* 105A** | BL105A | x | x | x | x | x | x | complete | NCBI database |
| ***B. longum* F8** | BIL | x | x | x | x | x | x | complete | NCBI database |
| ***B. longum* NCC 2705** | BL2705 | x | x | x | x | x | x | complete | NCBI database |
| ***B. longum* 157F** | BLlF | x | x | x | x | x | x | complete | NCBI database |
| ***B. longum* ATCC 15697** | BLIJ | x | x | x | x | x | x | complete | NCBI database |
| ***B. longum* KACC 91563** | BLNIAS | x | x | x | x | x | x | complete | NCBI database |
| ***B. longum* JCM1217** | BLLJ | x | x | x | x | x | x | complete | NCBI database |
| ***B. longum* CCUG 30698** | BBL306 | x | x | x | x | x | x | complete | NCBI database |
| ***B. longum* BXY01** | BXY01 | | | | | x | x | complete | NCBI database |
| ***B. longum* BT1** | BT1 | | | | | x | | complete | NCBI database |
| ***B. longum* BG7** | BG7 | | | | | x | x | complete | NCBI database |
| ***B. longum* E18** | AUYD01 | | | | | x | x | complete | NCBI database |
| ***B. longum* BT1** | | | | | | | x | draft | NCBI database |
| ***B. longum* EK3** | | | | | | | x | draft | NCBI database |
| ***B. longum* BIB1401242951** | | | | | | | x | draft | NCBI database |
| ***B. longum* BIB1401272845b** | | | | | | | x | draft | NCBI database |
| ***B. longum* BIC1401212621a** | | | | | | | x | draft | NCBI database |
| ***B. longum* BIC1401272845a** | | | | | | | x | draft | NCBI database |
| ***B. longum* BIC1401212621b** | | | | | | | x | draft | NCBI database |
| ***B. longum* BIC1401111250** | | | | | | | x | draft | NCBI database |
| ***B. longum* BIC1307292462** | | | | | | | x | draft | NCBI database |
| ***B. longum* BIC1206122787** | | | | | | | x | draft | NCBI database |
| ***B. longum* CMCC P001** | | | | | | | x | draft | NCBI database |
| ***B. longum* AGR 2137** | | | | | | | x | draft | NCBI database |
| ***B. longum* DSM 20211** | | | | | | | x | draft | NCBI database |
| ***B. longum* LMG 21814** | | | | | | | x | draft | NCBI database |
| ***B*. *longum* VMKB44** | | | | | | | x | draft | NCBI database |
| ***B. longum* BLO12** | | | | | | | x | draft | NCBI database |
| ***B. longum* 72B** | | | | | | | x | draft | NCBI database |
| ***B. longum*** 9 | | | | | | | x | draft | NCBI database |
| ***B. longum*** 7 | | | | | | | x | draft | NCBI database |
| ***B. longum* 17-1B** | | | | | | | x | draft | NCBI database |
| ***B. longum* 1-6B** | | | | | | | x | draft | NCBI database |
| ***B. longum* 44B** | | | | | | | x | draft | NCBI database |
| ***B. longum* 379** | | | | | | | x | draft | NCBI database |
| ***B. longum* 1-5B** | | | | | | | x | draft | NCBI database |
| ***B. longum* LMG 13197** | | | | | | | x | draft | NCBI database |
| ***B. longum* D2957** | | | | | | | x | draft | NCBI database |
| ***B. longum* CECT 7210** | | | | | | | x | draft | NCBI database |
| ***B. longum* CECT 7347** | | | | | | | x | draft | NCBI database |
| ***B. longum* MC-42** | | | | | | | x | draft | NCBI database |
| ***B. longum* CMW7750** | | | | | | | x | draft | NCBI database |
| ***B. longum* CCUG 52486** | | | | | | | x | draft | NCBI database |
| ***B. longum* EK13** | | | | | | | x | draft | NCBI database |
| ***B. longum* ATCC 55813** | | | | | | | x | draft | NCBI database |
| ***B. longum* 7-1B** | | | | | | | x | draft | NCBI database |
| ***B. longum* 35B** | | | | | | | x | draft | NCBI database |
| ***B. longum* 2-2B** | | | | | | | x | draft | NCBI database |
| ***B. longum* EK5** | | | | | | | x | draft | NCBI database |

**Table 6: EC annotation numbers of the in silica glycosyl hydrolases predicted.**

| **Enzyme family** | **GH family** | **EC numbers** |
|---|---|---|
| (Ara-f)(3)-Hypbeta-L-arabinobiosidase | GH121 | 3.2.1.187 |
| (Ara-f)(3)-Hypbeta-L-arabinobiosidase | GH43 | 3.2.1.187 |
| 1,4-alpha-glucanbranchingenzyme | GH13 | 2.4.1.18 |
| Alpha-amylase | GH13 | 3.2.1.1 |
| Alpha-D-xylosidexylohydrolase | GH31 | 3.2.1.177 |
| Alpha-galactosidase | GH36 | 3.2.1.22 |
| Alpha-galactosidase | GH27 | 3.2.1.22 |
| Alpha-glucosidase | GH13 | 3.2.1.20 |
| Alpha-L-fucosidase | GH95 | 3.2.1.51 |
| Alpha-mannosidase | GH38 | 3.2.1.24 |
| Amylosucrase | GH13 | 2.4.1.4 |
| Arabinanendo-1,5-alpha-L-arabinosidase | GH43 | 3.2.1.99 |
| Arabinogalactanendo-beta-1,4-galactanase | GH53 | 3.2.1.89 |
| Beta-glucosidase | GH1 | 3.2.1.38 |
| Beta-fructofuranosidase | GH32 | 3.2.1.26 |
| Beta-galactosidase | GH42 | 3.2.1.23 |
| Beta-galactosidase | GH2 | 3.2.1.23 |
| Beta-glucosidase | GH3 | 3.2.1.21 |
| Beta-glucosidase | GH1 | 3.2.1.21 |
| Beta-N-acetylhexosaminidase | GH3 | 3.2.1.52 |
| Beta-N-acetylhexosaminidase | GH20 | 3.2.1.52 |
| Cyclomaltodextrinase | GH13 | 3.2.1.54 |
| Dextransucrase | GH25 | 2.4.1.5 |
| Endo-1,4-beta-xylanase | GH5 | 3.2.1.8 |
| Endo-alpha-N-acetylgalactosaminidase | GH101 | 3.2.1.97 |
| Galactosylceramidase | GH59 | 3.2.1.46 |
| Gellanlyase | GH43 | 4.2.2.25 |
| Glucan1,3-beta-glucosidase | GH5 | 3.2.1.58 |
| Glucanendo-1,6-beta-glucosidase | GH30 | 3.2.1.75 |
| Isoamylase | GH13 | 3.2.1.68 |
| L-arabinofuranosidase | GH51 | 3.2.1.55 |
| L-arabinofuranosidase | GH43 | 3.2.1.55 |
| L-arabinofuranosidase | GH127 | 3.2.1.185 |
| Oligo-1,6-glucosidase | GH13 | 3.2.1.10 |
| Pullulanase | GH13 | 3.2.1.41 |
| Xylan1,4-beta-xylosidase | GH43 | 3.2.1.37 |

**Table 7. Carbohydrates used for in vitro assays.**

| **Carbohydrates** | **Company** | **Source** |
|---|---|---|
| 2'-O-Fucosyllactose | Glycom | |
| 3'-O-Fucosyllactose | Glycom | |
| Amylopectin | Fluka | Maize |
| Arabinan | Megazyme | Sugar Beet |
| Arabinogalactan | Megazyme | Larch Wood |
| L-Arabinose | Sigma | |
| Arabinoxylan (rye) | Megazyme | Rye Flour |
| Arabinoxylan (wheat) | Megazyme | Wheat Flour |
| FOS | | |
| Fucose | | |
| Galactan | Megazyme | Potato |
| D-Galactose | Carbosynth | |
| D-Glucose monohydrate | Sigma | |
| GaS | | |
| Inulin | Sigma | |
| α-Lactose monohydrate | Sigma | |
| Lacto-N-neotretaose | Glycom | |
| D-Mannose | Sigma | |
| Mucin | | |
| N-acetyl glucosamine | | |
| N-acetyl mannosamine | | |
| N-acetyl galactosamine | | |
| Pectic Galactan | Megazyme | Potato |
| Pectin | Sigma | Apple |
| Pullulan | | |
| Sialic acid | | |
| Starch | Sigma | |
| Sucrose | Sigma | |
| Xylo-oligosaccharide | | |
| P95 | Longlife | |
| Xylan | Megazyme | Beechwood |
| D-Xylose | Fluka | |

## Claims

1. A composition comprising a cohort of at least five isolated strains of *Bifidobacterium longum* selected from the group consisting of:
*Bifidobacterium longum* ssp *suis* APC1461 as deposited with the National Collection of Industrial and Marine Bacteria under the Accession No. NCIMB 42832 on 29 September 2017;
*Bifidobacterium longum* ssp *longum* APC1473 as deposited with the National Collection of Industrial and Marine Bacteria under the Accession No. NCIMB 42824 on 29 September 2017;
*Bifidobacterium longum* ssp *longum* APC1476 as deposited with the National Collection of Industrial and Marine Bacteria under the Accession No. NCIMB 42825 on 29 September 2017;
*Bifidobacterium longum* ssp *longum* APC1478 as deposited with the National Collection of Industrial and Marine Bacteria under the Accession No. NCIMB 42826 on 29 September 2017;
*Bifidobacterium longum* ssp *longum* APC1480 as deposited with the National Collection of Industrial and Marine Bacteria under the Accession No. NCIMB 42827 on 29 September 2017;
*Bifidobacterium longum* ssp *longum* APC1503 as deposited with the National Collection of Industrial and Marine Bacteria under the Accession No. NCIMB 42828 on 29 September 2017;
*Bifidobacterium longum* ssp *longum* APC293 as deposited with the National Collection of Industrial and Marine Bacteria under the Accession No. NCIMB 42830 on 29 September 2017;
*Bifidobacterium longum* ssp *longum* APC295 as deposited with the National Collection of Industrial and Marine Bacteria under the Accession No. NCIMB 42831 on 29 September 2017;
*Bifidobacterium longum* ssp *longum* APC1462 as deposited with the National Collection of Industrial and Marine Bacteria under the Accession No. NCIMB 42833 on 29 September 2017;
*Bifidobacterium longum* ssp *longum* APC1465 as deposited with the National Collection of Industrial and Marine Bacteria under the Accession No. NCIMB 42834 on 29 September 2017;
*Bifidobacterium longum* ssp *longum* APC1464 as deposited with the National Collection of Industrial and Marine Bacteria under the Accession No. NCIMB 42839 on 5 October 2017;
*Bifidobacterium longum* ssp *longum* APC1468 as deposited with the National Collection of Industrial and Marine Bacteria) under the Accession No. NCIMB 42840 on 5 October 2017; and
*Bifidobacterium longum* ssp *longum* APC1504 as deposited with the National Collection of Industrial and Marine Bacteria under the Accession No. NCIMB 42841 on 5 October 2017.

2. A composition according to Claim 1, in which the cohort of isolated strains includes at least one of:
*Bifidobacterium longum* ssp *longum* APC1478; and
*Bifidobacterium longum* ssp *longum* APC295.

3. A composition according to Claim 2, in which the cohort of strains includes:
*Bifidobacterium longum* ssp *longum* APC1478; and
*Bifidobacterium longum* ssp *longum* APC295.

4. A composition according to any of Claims 1 to 3, in which the cohort of strains includes
*Bifidobacterium longum* ssp *longum* APC1477.

5. A composition according to Claim 4, in which the cohort of strains includes:
*Bifidobacterium longum* ssp *longum* APC1478;
*Bifidobacterium longum* ssp *longum* APC295; and
*Bifidobacterium longum* ssp *longum* APC1477.

6. A composition according to any preceding claim, in which the cohort of isolated strains of *Bifidobacterium longum* together contain at least 60% of the total gene repertoire of the *Bifidobacterium longum* pangenome.

7. A composition according to Claim 6, in which the cohort of isolated strains comprises *Bifidobacterium longum* ssp *longum* APC1477 and at least 5 isolated strains of *Bifidobacterium longum* selected from the group of Claim 1 including *Bifidobacterium longum* ssp *longum* APC1478 and *Bifidobacterium longum* ssp *longum* APC295.

8. A composition according to any preceding Claim, in which the cohort of isolated strains comprises at least 6 isolated strains of *Bifidobacterium longum* selected from the group of Claim 1.

9. A composition according to any preceding Claim, in which the cohort of isolated strains comprises at least 8 isolated strains of *Bifidobacterium longum* selected from the group of Claim 1.

10. A composition according to any preceding Claim, in which the composition is an infant milk formula.

11. A composition according to any of Claims 1 to 10, for use in a method of treating an infant having a dysregulated microbiome.

12. A composition according to any of Claims 1 to 10, for use of Claim 11, in which the infant is selected from a pre-term infant, an infant born by caesarean section, and an infant having undergone or undergoing antibiotic therapy.

## Patentansprüche

1. Zusammensetzung, die eine Kohorte von mindestens fünf isolierten Stämmen von *Bifidobacterium longum* umfasst, die aus der Gruppe ausgewählt sind, die aus Folgenden besteht:
*Bifidobacterium longum* ssp *suis* APC1461, wie bei der National Collection of Industrial and Marine Bacteria unter der Zugangsnummer NCIMB 42832 am 29. September 2017 hinterlegt;
*Bifidobacterium longum* ssp *longum* APC1473, wie bei der National Collection of Industrial and Marine Bacteria unter der Zugangsnummer NCIMB 42824 am 29. September 2017 hinterlegt;
*Bifidobacterium longum* ssp *longum* APC1476, wie bei der National Collection of Industrial and Marine Bacteria unter der Zugangsnummer NCIMB 42825 am 29. September 2017 hinterlegt;
*Bifidobacterium longum* ssp *longum* APC1478, wie bei der National Collection of Industrial and Marine Bacteria unter der Zugangsnummer NCIMB 42826 am 29. September 2017 hinterlegt;
*Bifidobacterium longum* ssp *longum* APC1480, wie bei der National Collection of Industrial and Marine Bacteria unter der Zugangsnummer NCIMB 42827 am 29. September 2017 hinterlegt;
*Bifidobacterium longum* ssp *longum* APC1503, wie bei der National Collection of Industrial and Marine Bacteria unter der Zugangsnummer NCIMB 42828 am 29. September 2017 hinterlegt;
*Bifidobacterium longum* ssp *longum* APC293, wie bei der National Collection of Industrial and Marine Bacteria unter der Zugangsnummer NCIMB 42830 am 29. September 2017 hinterlegt;
*Bifidobacterium longum* ssp *longum* APC295, wie bei der National Collection of Industrial and Marine Bacteria unter der Zugangsnummer NCIMB 42831 am 29. September 2017 hinterlegt;
*Bifidobacterium longum* ssp *longum* APC1462, wie bei der National Collection of Industrial and Marine Bacteria unter der Zugangsnummer NCIMB 42833 am 29. September 2017 hinterlegt;
*Bifidobacterium longum* ssp *longum* APC1465, wie bei der National Collection of Industrial and Marine Bacteria unter der Zugangsnummer NCIMB 42834 am 29. September 2017 hinterlegt;
*Bifidobacterium longum* ssp *longum* APC1464, wie bei der National Collection of Industrial and Marine Bacteria unter der Zugangsnummer NCIMB 42839 am 5. Oktober 2017 hinterlegt;
*Bifidobacterium longum* ssp *longum* APC1468, wie bei der National Collection of Industrial and Marine Bacteria unter der Zugangsnummer NCIMB 42840 am 5. Oktober 2017 hinterlegt; und
*Bifidobacterium longum* ssp *longum* APC1504, wie bei der National Collection of Industrial and Marine Bacteria unter der Zugangsnummer NCIMB 42841 am 5. Oktober 2017 hinterlegt.

2. Zusammensetzung nach Anspruch 1, in der die Kohorte von isolierten Stämmen mindestens eines von Folgenden einschließt:
*Bifidobacterium longum* ssp *longum* APC1478; und
*Bifidobacterium longum* ssp *longum* APC295.

3. Zusammensetzung nach Anspruch 2, in der die Kohorte von Stämmen Folgendes einschließt:
*Bifidobacterium longum* ssp *longum* APC1478; und
*Bifidobacterium longum* ssp *longum* APC295.

4. Zusammensetzung nach einem der Ansprüche 1 bis 3, in der die Kohorte von Stämmen
*Bifidobacterium longum* ssp *longum* APC1477 einschließt.

5. Zusammensetzung nach Anspruch 4, in der die Kohorte von Stämmen Folgendes einschließt:
*Bifidobacterium longum* ssp *longum* APC1478;
*Bifidobacterium longum* ssp *longum* APC295; und
*Bifidobacterium longum* ssp *longum* APC1477.

6. Zusammensetzung nach einem vorstehenden Anspruch, in der die Kohorte von isolierten Stämmen von *Bifidobacterium longum* zusammen mindestens 60 % des gesamten Genrepertoire des Pangenoms von *Bifidobacterium longum* enthält.

7. Zusammensetzung nach Anspruch 6, in der die Kohorte von isolierten Stämmen *Bifidobacterium longum* ssp *longum* APC1477 und mindestens 5 isolierte Stämme von *Bifidobacterium longum* umfasst, die aus der Gruppe von Anspruch 1 ausgewählt sind, die *Bifidobacterium longum* ssp *longum* APC1478 und *Bifidobacterium longum* ssp *longum* APC295 einschließt.

8. Zusammensetzung nach einem vorstehenden Anspruch, in der die Kohorte von isolierten Stämmen mindestens 6 isolierte Stämme von *Bifidobacterium longum* umfasst, die aus der Gruppe von Anspruch 1 ausgewählt sind.

9. Zusammensetzung nach einem vorstehenden Anspruch, in der die Kohorte von isolierten Stämmen mindestens 8 isolierte Stämme von *Bifidobacterium longum* umfasst, die aus der Gruppe von Anspruch 1 ausgewählt sind.

10. Zusammensetzung nach einem vorstehenden Anspruch, in der die Zusammensetzung eine Säuglingsmilchnahrung ist.

11. Zusammensetzung nach einem der Ansprüche 1 bis 10 für die Verwendung in einem Verfahren zur Behandlung eines Säuglings, der ein dysreguliertes Mikrobiom aufweist.

12. Zusammensetzung nach einem der Ansprüche 1 bis 10 für die Verwendung nach Anspruch 11, in der der Säugling aus einem frühgeborenen Säugling, einem durch Kaiserschnitt geborenen Säugling und einem Säugling, der Antibiotikatherapie unterzogen wurde oder wird, ausgewählt ist.

## Revendications

1. Composition comprenant une cohorte d'au moins cinq souches isolées de *Bifidobacterium longum* choisies dans le groupe constitué par :
*Bifidobacterium longum* ssp *suis* APC1461 telle que déposée auprès de la Collection Nationale de Bactéries Industrielles et Marines sous le numéro d'Accès NCIMB 42832 le 29 septembre 2017 ;
*Bifidobacterium longum* ssp *longum* APC1473 telle que déposée auprès de la Collection Nationale de Bactéries Industrielles et Marines sous le numéro d'Accès NCIMB 42824 le 29 septembre 2017 ;
*Bifidobacterium longum* ssp *longum* APC1476 telle que déposée auprès de la Collection Nationale de Bactéries Industrielles et Marines sous le numéro d'Accès NCIMB 42825 le 29 septembre 2017 ;
*Bifidobacterium longum* ssp *longum* APC1478 telle que déposée auprès de la Collection Nationale de Bactéries Industrielles et Marines sous le numéro d'Accès NCIMB 42826 le 29 septembre 2017 ;
*Bifidobacterium longum* ssp *longum* APC1480 telle que déposée auprès de la Collection Nationale de Bactéries Industrielles et Marines sous le numéro d'Accès NCIMB 42827 le 29 septembre 2017 ;
*Bifidobacterium longum* ssp *longum* APC1503 telle que déposée auprès de la Collection Nationale de Bactéries Industrielles et Marines sous le numéro d'Accès NCIMB 42828 le 29 septembre 2017 ;
*Bifidobacterium longum* ssp *longum* APC293 telle que déposée auprès de la Collection Nationale de Bactéries Industrielles et Marines sous le numéro d'Accès NCIMB 42830 le 29 septembre 2017 ;
*Bifidobacterium longum* ssp *longum* APC295 telle que déposée auprès de la Collection Nationale de Bactéries Industrielles et Marines sous le numéro d'Accès NCIMB 42831 le 29 septembre 2017 ;
*Bifidobacterium longum* ssp *longum* APC1462 telle que déposée auprès de la Collection Nationale de Bactéries Industrielles et Marines sous le numéro d'Accès NCIMB 42833 le 29 septembre 2017 ;
*Bifidobacterium longum* ssp *longum* APC1465 telle que déposée auprès de la Collection Nationale de Bactéries Industrielles et Marines sous le numéro d'Accès NCIMB 42834 le 29 septembre 2017 ;
*Bifidobacterium longum* ssp *longum* APC1464 telle que déposée auprès de la Collection Nationale des Bactéries Industrielles et Marines sous le numéro d'Accès NCIMB 42839 le 5 octobre 2017 ;
*Bifidobacterium longum* ssp *longum* APC1468 telle que déposée auprès de la Collection Nationale de Bactéries Industrielles et Marines sous le numéro d'Accès NCIMB 42840 le 5 octobre 2017 ; et
*Bifidobacterium longum* ssp *longum* APC1504 telle que déposée auprès de la Collection Nationale de Bactéries Industrielles et Marines sous le numéro d'Accès NCIMB 42841 le 5 octobre 2017.

2. Composition selon la revendication 1, dans laquelle la cohorte de souches isolées comporte au moins l'une parmi :
*Bifidobacterium longum* ssp *longum* APC1478 ; et
*Bifidobacterium longum* ssp *longum* APC295.

3. Composition selon la revendication 2, dans laquelle la cohorte de souches comporte :
*Bifidobacterium longum* ssp *longum* APC1478 ; et
*Bifidobacterium longum* ssp *longum* APC295.

4. Composition selon l'une quelconque des revendications 1 à 3, dans laquelle la cohorte de souches comporte :
*Bifidobacterium longum* ssp *longum* APC1477.

5. Composition selon la revendication 4, dans laquelle la cohorte de souches comporte :
*Bifidobacterium longum* ssp *longum* APC1478 ;
*Bifidobacterium longum* ssp *longum* APC295 ; et
*Bifidobacterium longum* ssp *longum* APC1477.

6. Composition selon l'une quelconque des revendications précédentes, dans laquelle la cohorte de souches isolées de *Bifidobacterium longum* contient dans son ensemble au moins 60% du répertoire total de gènes du pangénome de *Bifidobacterium longum.*

7. Composition selon la revendication 6, dans laquelle la cohorte de souches isolées comprend *Bifidobacterium longum* ssp *longum* APC1477 et au moins 5 souches isolées de *Bifidobacterium longum* choisies dans le groupe selon la revendication 1, y compris *Bifidobacterium longum* ssp *longum* APC1478 et *Bifidobacterium longum* ssp *longum* APC295.

8. Composition selon l'une quelconque des revendications précédentes, dans laquelle la cohorte de souches isolées comprend au moins 6 souches isolées de *Bifidobacterium longum* choisies dans le groupe selon la revendication 1.

9. Composition selon l'une quelconque des revendications précédentes, dans laquelle la cohorte de souches isolées comprend au moins 8 souches isolées de *Bifidobacterium longum* choisies dans le groupe selon la revendication 1.

10. Composition selon l'une quelconque des revendications précédentes, la composition étant un lait maternisé.

11. Composition selon l'une quelconque des revendications 1 à 10, pour une utilisation dans une méthode de traitement d'un nourrisson présentant un microbiome dysrégulé.

12. Composition selon l'une quelconque des revendications 1 à 10, pour une utilisation selon la revendication 11, le nourrisson étant choisi parmi un nourrisson prématuré, un nourrisson né par césarienne, et un nourrisson ayant fait l'objet ou faisant l'objet d'une antibiothérapie.
